# EUROPEAN PATENT SPECIFICATION

(11) **EP 1 240 338 B1**
(45) Date of publication and mention of the grant of the patent: **09.06.2004**
(21) Application number: 00993631.1
(22) Date of filing: 22.12.2000
(51) Int. Cl.: C12N 15/63, C12N 15/70, C12N 9/02, C07K 14/415, C12N 15/29, C12N 15/53, C12P 21/00, C12Q 1/68, G01N 33/53, A61K 38/00

(54) **PRODUCTION OF HETEROLOGOUS PROTEINS**
HERSTELLUNG VON HETERLOGE PROTEINE
PRODUCTION DE PROTEINES HETEROLOGUES

(30) Priority: 24.12.1999 GB 9930480
(43) Date of publication of application: 18.09.2002
(73) Proprietor: The University of Wales, Aberystwyth SY23 2AX (GB)
(72) Inventor: KADERBHAI, Mustak Ali, Waum Fawr, Aberystwyth SY23 3PP (GB); KELLY, Steven, Lewis, Aberystwyth SY23 3HR (GB); LAMB, David Christopher, Aberystwyth SY23 3QQ (GB)
(74) Representative: Downing, Michael Philip
(86) International application number: PCT/EP2000/013352
(87) International publication number: WO 2001/048225

(56) References cited:
- WO-A-89/11789
- WO-A-98/14604
- PRITCHARD MICHAEL P ET AL: "A general strategy for the expression of recombinant human cytochrome P450s in Escherichia coli using bacterial signal peptides: Expression of CYP3A4, CYP2A6, and CYP2E1." ARCHIVES OF BIOCHEMISTRY AND BIOPHYSICS, vol. 345, no. 2, 1997, pages 342-354, XP002171497 ISSN: 0003-9861 cited in the application
- NIELSEN H ET AL: "IDENTIFICATION OF PROKARYOTIC AND EUKARYOTIC SIGNAL PEPTIDES AND PREDICTION OF THEIR CLEAVAGE SITES" PROTEIN ENGINEERING,GB,OXFORD UNIVERSITY PRESS, SURREY, vol. 10, no. 1, 1997, pages 1-6, XP002072638 ISSN: 0269-2139 cited in the application
- KEEGSTRA K: "Transport and routing of proteins into chloroplasts" CELL,CELL PRESS, CAMBRIDGE, NA,US, vol. 56, 27 January 1989 (1989-01-27), pages 247-253, XP002122099 ISSN: 0092-8674 cited in the application
- DABNEY-SMITH CAROLE ET AL: "The C terminus of a chloroplast precursor modulates its interaction with the translocation apparatus and PIRAC." JOURNAL OF BIOLOGICAL CHEMISTRY, vol. 274, no. 45, 5 November 1999 (1999-11-05), pages 32351-32359, XP002171499 ISSN: 0021-9258
- PINNADUWAGE PURNIMA ET AL: "In vitro interaction between a chloroplast transit peptide and chloroplast outer envelope lipids is sequence-specific and lipid class-dependent." JOURNAL OF BIOLOGICAL CHEMISTRY, vol. 271, no. 51, 1996, pages 32907-32915, XP002171500 ISSN: 0021-9258
- LIU YAN-YUN ET AL., : "A chloroplast envelope-transfer sequence functions as an export signal in Escherichia coli" FEBS LETT., vol. 469, no. 1, 3 March 2000 (2000-03-03), pages 61-66, XP002171498 cited in the application
- LIU YAN-YUN ET AL: "A mammalian cytochrome fused to a chloroplast transit peptide is a functional haemoprotein and is imported into isolated chloroplasts." BIOCHEMICAL JOURNAL, vol. 351, no. 2, 15 October 2000 (2000-10-15), pages 377-384, XP002171501 ISSN: 0264-6021 cited in the application

## Description

The present invention relates to processes for the production of heterologous proteins in Gram-negative prokaryotes (such as *Escherichia coli),* and in particular to processes in which the expressed heterologous proteins are targeted to the outer membrane and/or periplasm of the bacterial cell after export through the inner membrane (and, where targeting to the outer membrane is desired, across the periplasmic space). The invention also relates to vectors suitable for use in such processes and to the expressed polypeptides *per se,* and in particular to a vector for expressing a heterologous gene encoding a polypeptide of interest in a Gram-negative prokaryote and targeting the expressed polypeptide to the outer membrane or periplasm thereof.

### Background of the Invention

Many proteins of industrial and/or medical importance are difficult or expensive to isolate in useful quantities from their natural sources. One known solution to this problem involves the isolation (or synthesis) of a gene encoding the protein of interest and its expression in a heterologous host cell where it can be produced at high levels and easily extracted.

Many different host cells have been used for such *heterologous gene expression.* They include eukaryotic cells, for example plant cells, animal cells (such as CHO cells) and yeast cells. However, over the past twenty years prokaryotic cells, and particularly *Escherichia coli,* have been recognized as among the most tractable hosts for the expression of a wide range of commercially important proteins and this technology is now well-established (see Ads (1990), Methods Enzymol. 182, 93-112).

An expression system based on *E. coli* (or other Gram-negative prokaryotic cell) can be considered to comprise five different compartments: the cytoplasm, the inner membrane (IM), the periplasm (PP), the outer membrane (OM) and the extracellular medium. Depending on the nature of the protein being expressed, yield and/or activity may depend on the compartment to which the protein is targeted, and much research has focussed on the development of techniques for selectively expressing recombinant proteins in different compartments.

Despite the fact that the production of recombinant proteins in *Escherichia coli* is a well-established technology, not all foreign genes are expressible in a facile manner to yield biologically active products. A major limiting factor is the formation of inclusion bodies, which although easing their isolation, necessitates the renaturation of the product Secretion or export of the foreign proteins into the less hostile periplasm offers an amenable approach for the generation of correctly folded molecules in a suitable oxidising environment and for their isolation in a concentrated state. The simpler protein composition of the periplasm over that of the cytoplasm can offer a significantly purified product in the osmotic shock supernatants. By way of export, a defined and retrievable amino-terminus can also be introduced between the signal and the passenger part of the protein.

However, at present there are no techniques available for targeting heterologous proteins expressed in Gram-negative bacterial cells to the OM. The development of such a technique would be of great importance, since many proteins (particularly the endogenous membrane proteins) might be expressible in an active conformation and at high yields only in this compartment. While techniques are known for targeting expressed proteins to the PP ( a basic theme involves appendage of the 21 residue secretory signal of alkaline phosphatase of *E. coli* at the N-terminus), problems may be encountered: the protein may not be fully active after translocation through the IM, may be recoverable only at poor yields, may be incompletely processed and/or may not assume the native conformation. Thus, an alternative means for targeting expressed proteins to the PP would be of considerable value.

### Summary of the Invention

It has now been discovered that a certain class of signalling elements derived from the chloroplast translocation pathway in higher plants can serve as an OM and/or PP targeting signal in Gram-negative bacteria such as *E. coli.* These elements are discussed in more detail below.

Chloroplast biogenesis in plants is dependent upon the co-ordinated activities of two independent genetic systems localised in the chloroplast and the nucleus (see Cline and Henry (1996), Annu. Rev. Cell Dev. Biol. 12, 1-26). The vast constituent chloroplast proteins are encoded by the nuclear genes and are cytoplasmically-synthesised as precursor forms which contain N-terminal extensions known as transit peptides. The transit peptide is instrumental for specific recognition of the chloroplast surface and in mediating the post-translational translocation of pre-proteins across the chloroplast envelope and thence to the various different sub-compartments within the chloroplast (e.g. stroma, thylakoid and thylakoid membrane).

At least two distinct functional domains have been identified in chloroplast transit peptides: the stromal targeting domain (STD) and the lumen targeting domain (LTD). STDs govern access to the general import pathway and are both necessary and sufficient for import of the passenger protein to the stroma.

Strornal protein precursors possess transit peptides that contain only an STD, whereas thylakoid lumenal protein precursors have both an STD and an LTD.

STDs range in size from about 30 to 120 residues and are rich in hydroxylated residues and deficient in acidic residues. They tend to share several compositional motifs: an amino terminal 10- 15 residues devoid of Gly, Pro and charged residues; a variable middle region rich in Ser, Thr, Lys and Arg; and a carboxy-proximal region with loosely conserved sequence {Ile/Val-x-Ala/Cys*Ala) for proteolytic processing. However, there are no extensive blocks of sequence conservation, nor any conserved secondary structural motifs. Theoretical analyses suggest that STDs adopt predominantly random coil conformations.

According to the present invention there is provided a vector for expressing a heterologous gene encoding a polypeptide of interest in a Gram-negative prokaryote and targeting the expressed polypeptide to the outer membrane and/or periplasmic space thereof, the vector comprising nucleic acid encoding a stromal targeting domain (STD).

Any suitable STD may be used according to the invention. The STD may form part of a chloroplast transit peptide, or may be an isolated domain thereof. The STD may be generated synthetically (e.g. by solid phase synthesis) or by modification of a clone of a naturally occurring transit peptide. The STD may be a mutant STD in which one or more nucleotides have been added, substituted or deleted. Those skilled in the art will be able to determine, by routine trial and error, whether mutation of any given STD is required in order to optimize expression and/or targeting.

Any of a wide range of polypeptides of interest may be expressed and targeted according to the invention. Particularly preferred are haemoproteins, particularly members of the cytochrome P-450 superfamily of enzymes. These enzymes undertake wide ranging stereo and regiospecific biotransformations of xenobiotics as well as participating in the biosynthesis of important endogenous cellular constituents and secondary metabolises. Knowledge of these enzymes is critical to an understanding of drug metabolism and drug discovery and use can be made of these enzymes in biotransformations of industrial chemicals, natural products, pollutants, chemical libraries as well as inhibition studies for new bioactive molecules. There is therefore a great need to overproduce these haemoproteins in functional forms by recombinant techniques and in particularly preferred embodiments the invention finds application in the expression and targeting of these proteins. The proteins so produced may then be used, for example, in:
(a) screening of bioactive molecules (e.g. drugs);
(b) biotransformations;
(c) bioremediation;
(d) assay of bioactive molecules (e.g. drugs)

Examples of haemoproteins which may be produced according to the invention include members of the cytochrome P450 (CYP) families 1,2,3 and 4, but also all other human cytochromes P450 and those of other Kingdoms of Life. Besides cytochrome P450 enzymes, also other membrane proteins resident in the endoplasmic reticulum including associated electron donors and enzymes of xenobiotic Phase I and Phase II metabolism, for example, flavin monooxygenases, glutathione transferases, epoxide hydrolase can also be targets for production. Also, soluble enzymes may be targeted in the same way, including soluble cytochromes P450 as are found extensively in bacteria, and other engineered versions of membrane-bound enzymes which can be targeted to different compartments as soluble derivatives. Experiments demonstrating production of >4µmol/L culture for active membrane-bound and soluble cytochrome P450 have been achieved in this way.

The nature of the vector is also not critical to the invention - any suitable vector may be used, including plasmid, cosmid, bacteriophage, transposon, minichromosome, liposome or mechanical carrier. Particularly preferred are vectors which comprise nucleic acid encoding a polypeptide of interest operably linked to the nucleic acid encoding the STD. However, those skilled in the art will appreciate that the vectors of the invention have general utility in the expression of a wide range of proteins of interest, and for most applications may be conveniently provided in a form in which they are "empty" of nucleic acid encoding a protein of interest and so ready to accept the insertion of any nucleic acid of interest

As used herein, the term "operably linked" refers to a condition in which portions of a linear DNA sequence are capable of influencing the activity of other portions of the same linear DNA sequence. For example, nucleic acid encoding an STD is operably linked to nucleic acid encoding a polypeptide of interest if the linked nucleic acid sequences are expressed as a pre-polypeptide and targeted to the OM by dint of the activity of the STD. Similarly, a signal sequence (such as a periplasmic signal sequence) is operably linked to nucleic acid encoding a polypeptide if it is expressed as a precursor which participates in the secretion of the polypeptide; a promoter is operably linked to a coding sequence if it controls the transcription of the sequence; a ribosome binding site is operably linked to a coding sequence if it is positioned so as to permit translation.

The vector preferably further comprises prokaryotic expression elements, for example for directing expression in *Escherichia coli.* However, in some embodiments the vector may be configured (e.g. by the provision of targeting sequence(s) homologous to endogenous DNA sequences in the host) to exploit endogenous expression elements in the host which become operably linked to the relevant portions of the vector after introduction into the host cell (and after, for example, integration into the chromosome or into a resident plasmid). In such cases it is not necessary that the expression elements be incorporated into the vector itself

The expression element or elements provide for expression of the protein of interest at suitable levels and at convenient times. Any of a wide variety of expression elements may be used, and the expression element or elements may for example be selected from promoters, operators and ribosome binding sites. The element(s) may be regulatable, for example being inducible (via the addition of an inducer). For example, *lac-*based expression elements may be induced by the addition of IPTG, while *trp*-based elements may be induced by starvation for tryptophan.

The vector may further comprise a positive selectable marker and/or a negative selectable marker. The use of a positive selectable marker facilitates the selection and/or identification of cells containing the vector.

The vector may further comprise nucleic acid encoding a periplasmic signal sequence. This can increase the yield of active protein. Without wishing to be bound by any theory, it is possible that the use of periplasmic signal sequences can increase the rate at which the heterologous protein is cleared from the cytoplasm (in which compartment it might be relatively labile). In such embodiments, the signal sequence may be the bacterial alkaline phosphatase signal sequence. Preferably, the nucleic acid encoding the signal sequence is located upstream of that encoding the STD.

The vector may conveniently further comprise a multiple cloning site for inserting a gene encoding a polypeptide of interest into the vector. This greatly simplifies the task of subeloning genes of interest into the vector in the correct reading frame. Any of a wide selection of multiple cloning sites may be used.

Preferably, the vector further comprises nucleic acid encoding one or more selectable marker(s) and/or reporter elements. The vector may also comprise one or more prokaryotic origin(s) of replication. Particularly preferred for some applications are shuttle plasmids which have origins of replication which are functional in two or more different species of host cell (e.g. in yeast and *E. coli,* or in *E. coli and Bacillus subtilis).*

In another aspect, the invention relates to a prokaryotic host cell comprising the vector of the invention. Any suitable host cell may be used, including *Escherichia coli and Salmonella typhimurium.*

The cell of the invention finds particular utility when present in a composition as an inoculum. The composition preferably further comprises a carrier, for example a cryoprotective agent. Suitable cryoprotective agents include glycerol.

In another aspect the invention relates to a process for producing a polypeptide of interest comprising the steps of: (a) culturing the host cell of the invention; (b) harvesting the cultured cells; (c) fractionating the harvested cells to provide a fraction enriched in outer membranes; and (d) isolating the polypeptide of interest from the outer membrane fraction.

In a further aspect, the invention contemplates a process for producing a membrane-bound polypeptide of interest comprising the steps of.. (a) culturing the host cell of the invention; (b) harvesting the cultured cells; (c) fractionating the harvested cells to provide a fraction enriched in outer membranes containing the membrane-bound polypeptide of interest.

In the processes of the invention, step (a) may comprise inoculating a growth medium with the composition of the invention. Preferably, the processes of the invention comprise the preliminary step of introducing the vector of the invention into a Gram-negative prokaryote (for example, *Escherichia coli)* to provide a host cell of the invention. The vector may be a plasmid and is preferably introduced into the host cell by transformation.

In particularly preferred embodiments, the process of the invention is an industrial fermentation. In such embodiments, the cells may be grown in continuous or batch culture (e.g. in a chemostat) and in high volumes (for example, 20 litres or more).

The invention also contemplates a polypeptide obtainable by the process of the invention.

The invention will now be described by reference to particular examples. The examples are purely illustrative and are not intended to be limiting in any way.

### Brief Description of the Drawings

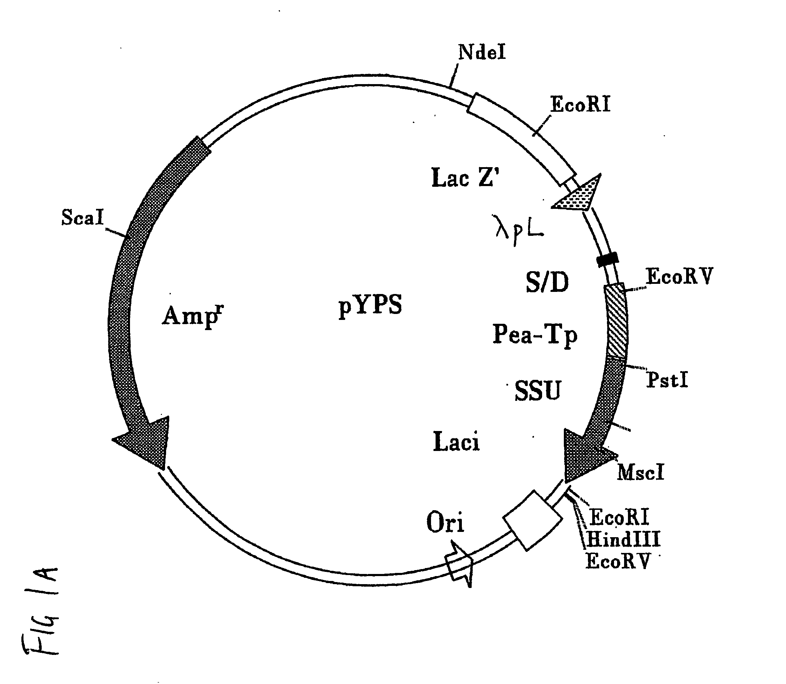

Figure 1 A shows the PYPS plasmid in which the upstream *PrSSU* segment was modified by replacing the *Hind*III*-Pvu*II deletion with a stretch of synthetic DNA duplex incorporating a ribosome binding site and an intervening sequence designed on *Pseudomonas putida* P450^{*cam*}*.* The modified gene was introduced into a region between the thermoregulated λP_{L} promoter and downstream mammalian cytochrome b₅ in p λ-lcyt.

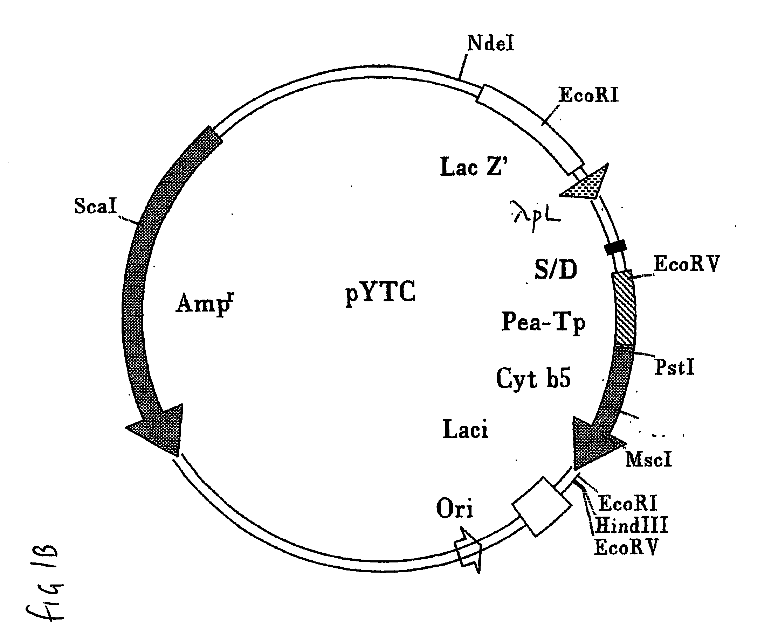

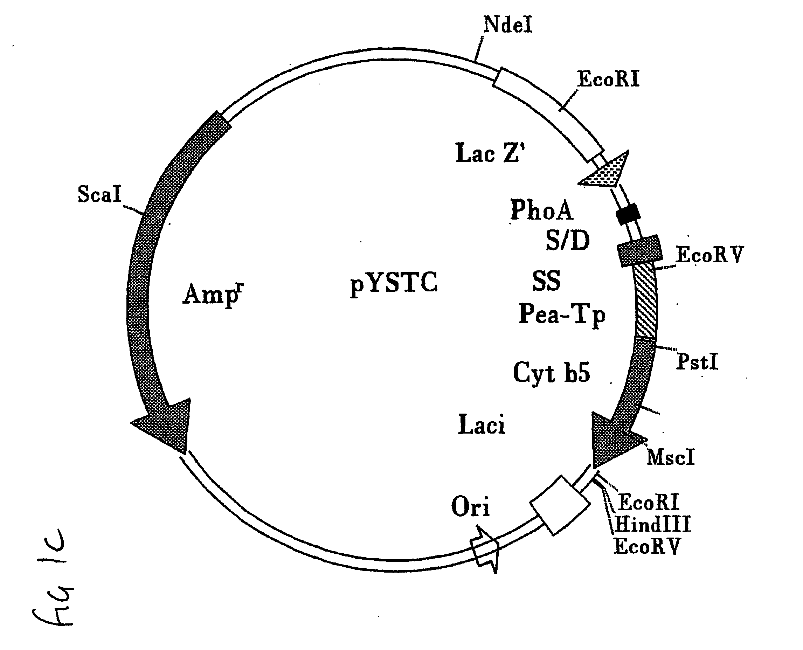

Figure 1B shows a plasmid construct comprising a *SphI-Ndel* deletion in PYPS replaced by the cytochrome b₅ gene carrying an engineered *Sph*I site at the N-terminus.
Figure 1C shows a plasmid construct comprising a *Sph*I*-Nde*I deletion in PYPS replaced by the cytochrome b₅ gene carrying an engineered *Sph*I site at the N-terminus, together with a periplasmic signal sequence (SS).

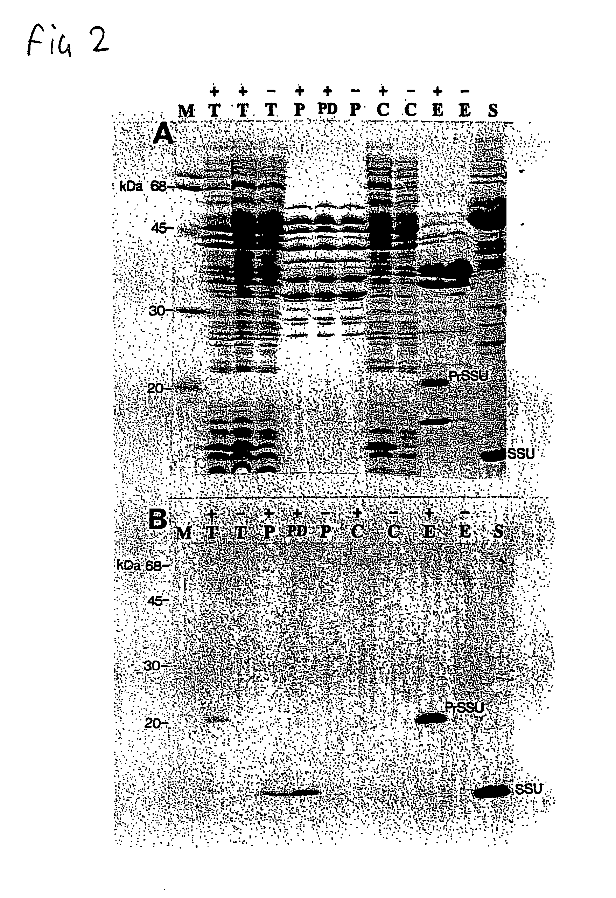

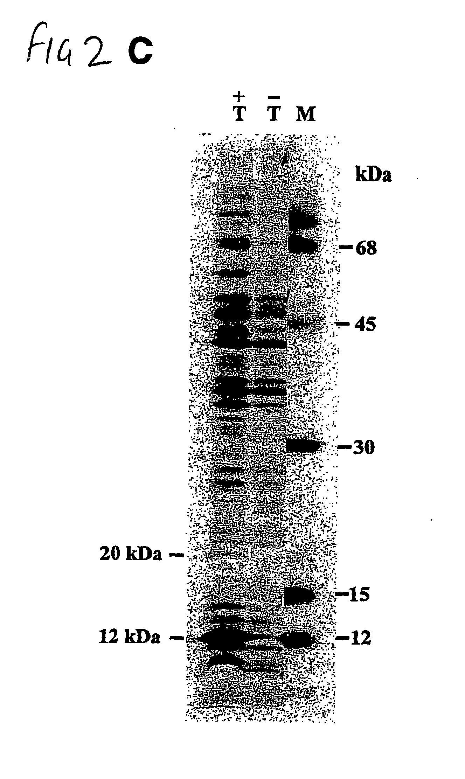

Figure 2 shows the subcellular localisation of PrSSU. In Figure 2, the identifiers are as follows:(A) Coomassie blue-stained, (B) Western blot of (A) probed with anti-pea SSU serum. M, Molecular weight marker proteins; T, total bacterial proteins; P, periplasmic fraction; PD, P after DEAE Sepharose CL-6B chromatography; C, cytosolic fraction; E, envelope membrane fraction. S, pea chloroplast stromal fraction. (+) and (-) denote proteins derived from 4h-thermoinduced or non-induced cells. (C) total cellular fraction separated on gradient polyacrylamide gel.

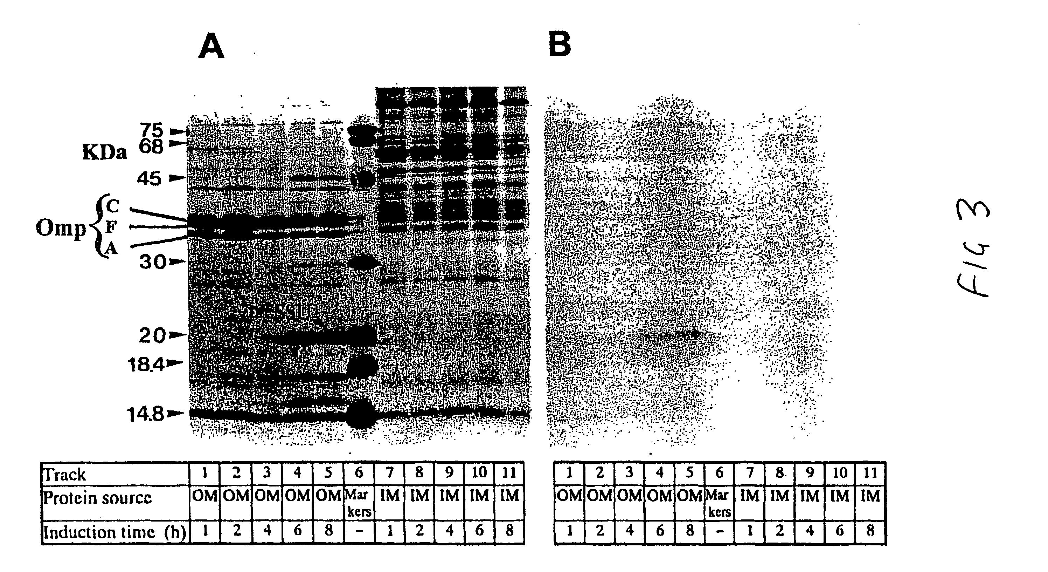

Figure 3 shows the immuno-electrophoretic localisation of PrSSU in isolated IM and OM. Envelope fractions of *E. coli* PYPS cells subfractionated into IM and OM. (A) Coomassic blue-stained, (B) Western of a comparable gel, shown in A, probed with anti-pea SSU serum.

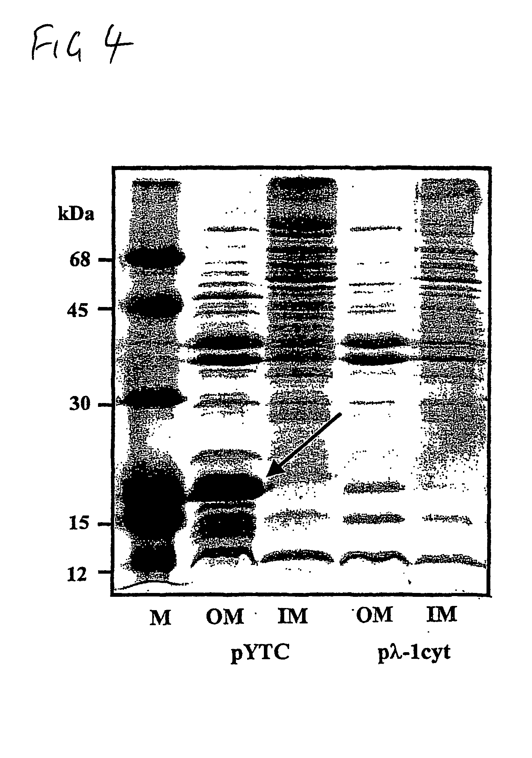

Figure 4 shows an SDS-PAGE run illustrating that the transit peptide-cytochrome b₅ is targeted to the OM. The proteins from *E. coli pλ-*1cyt controlled and pYTC were analysed by SDS-PAGE as described in the text below. The arrowheads show the position of the chimaeric transit peptide-cytochrome b₅ protein. M, marker proteins.

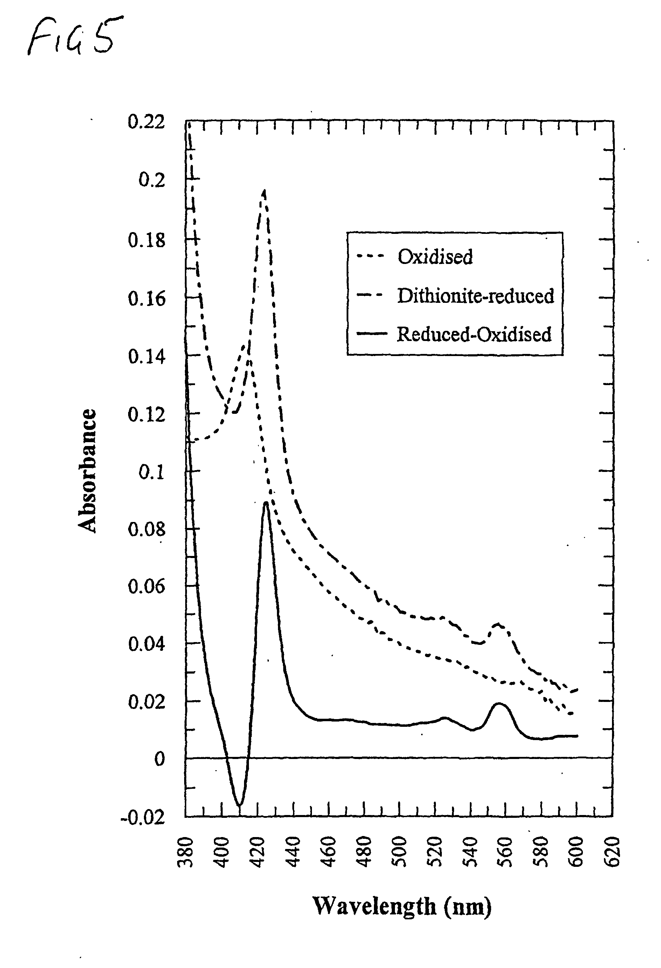

Figure 5 shows the spectral characteristics of isolated OM from *E. coli* PYTC expressing transit peptide- cytochrome b₅ fusion protein. The OM suspended at 75 µg protein/ml in 25 mM Tris-aeetate (pH.8) were scanned against the isolated OM from E. *coli* pAF (control) at a comparable concentration.

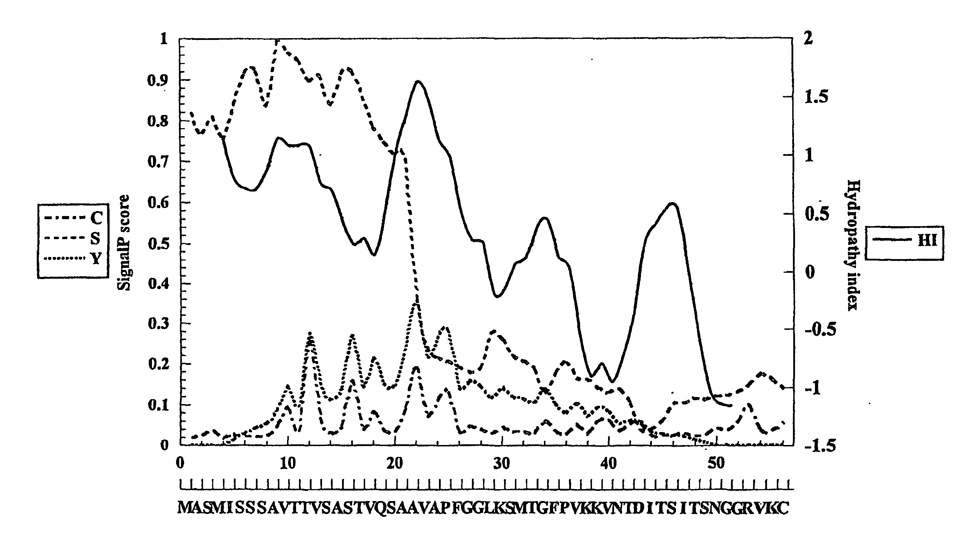

Figure 6 shows the predicted hydrophobic character on and signal sequence-like element in pea transit peptide. The hydropathy indices were calculated using Kyte and Doolittle algorithms (Kyte and Doolittle (1982), J. Mol. Biol. 157, 105-132), with a window setting (10% linear weighting with respect to the window centre) of 9 residues. The signal peptide score (S-score) and the combined cleavage site score (Y-score) were obtained using the Signalp prediction program of Nielsen *et aL* (1997), Protein Engineering 10, 1-6.

### Detailed Description of the Preferred Embodiments

### Example 1

### Expression and targeting of PrSSU to the OM in Escherichia coli

The precursor of the small subunit of RUBISCO (ribulose-1,5-bisphosphate carboxylase/oxygenase) (PrSSU) was expressed in *E. coli* using the tightly-regulated gP_{L} promoter. *E. Coli N4830-1* cultures, pre-grown in Luria broth (ampicillin 75mglml) at 30°C to an O.D. = 0.6 units, were thermo-induced at 39±1°C for durations specified elsewhere. *E. coli* subcellular fractionations were performed as described previously (Karim *et al.* (1993) Bio-Technology 11, 612-617). Proteins were analysed by SDS electrophoresis using either 12-18% gradient of polyacrylamide gels with sample loadings ranging from 50 to 100µg per lane. For Western blots, eletrophoresed proteins were transferred from an unstained gel into nitrocellulose sheets and the rabbit anti-pea mature small subunit of RUBISCO (SSU) reactive components detected by activity staining with horse-radish peroxidase conjugated to goat anti-rabbit IgG. The membrane-associated PrSSU, isolated by electroelution, was microsequenced by Edman degradation (Alta Laboratories, Birmingham University). The periplasmic anti-SSU reactive 14 kDa was isolated by electroelution following filtration of a ten-fold concentrated periplasmic fraction through an Amicon 30 filter unit. For immunoelectron microscopy, prefixed ultrathin sections *of E. coli* were treated with 0.5% bovine serum albumin, 0.2% gelatin in phosphate-buffered saline to block non-specific binding. After incubation with affinity-purified anti-SSU antibodies (1:500) the sections were extensively washed and labelled with 10nm protein A-coupled colloidal gold particles, essentially as recommended by Biocell conjugates (UK). Post fixation in osmium tetroxide, the sections were stained with 2% uranyl acetate and lead citrate (18) and examined using a Jeol JEM-100 CX transmission electron microscope at 100kV.

The 5'-proximally-modified PrSSU cDNA, containing an optimised ribosomal binding site and a choice of codons ideal for expression in *E. coli* was placed under the control of the thermoinducible λP_{L} promoter in the derivative plasmid pYPS (Figure 1A). The tandemly co-expressed cytochrome b₅ gene, placed downstream of *prSSU,* aided identification and isolation of the clone that expressed PrSSU through the pink reporter system (Kaderbhai *et al.* (1992) DNA and Cell Biology 11, 567-577). Thermoinduction of *E. coli* pYPS directed the synthesis of two proteins of 20 and 12 kDa (Figure 2). The latter, identified as the co-expressed cytochrome b₅ (see below), constituted approximately 9% of the total cellular protein (Figure 2A, cf lanes T+ and T-). Whereas the former appeared to represent a significantly smaller amount (∼ 1 %) of the total protein, its detection by Coomassic blue staining proved possible only when the total cellular fraction was separated on a (15-18%) gradient polyacrylamide gel that provided higher resolving capability (Figure 2C). The total cellular polypeptide profile, probed with anti-pea SSU antibodies, signalled cross-reactivity against the 20 kDa induced band and, to a lesser extent, with another 14 kDa protein (Figure 2B, cf lanes T+ and T-). An electroeuted preparation of the 20 kDa recombinant protein (see Materials and Methods) was subjected to 35 rounds of automated Edman degradations. This yielded an N-terminal sequence which was identical to that deduced from the nueleotide sequence of the PrSSU cDNA, except for the absence of the initiator methionine and the -15 arginine residue in the transit peptide. The absence of the formylmethionine initiator suggested that the PrSSU was processed in accordance with the substrate specificity of the cytoplasmic methionine aminopeptidase of *E. coli* (Hirel *et al (1989)* PNAS 86, 8247-8251).

To decipher the sub-cellular location of the PrSSU and the anti-SSU reactive 14kDa proteins, thermo-induced and non-induced *E. coli* pYPS cells were subfractionated into the periplasmic, cytoplasmic and envelope fractions. The effective separation of the bacterial compartments was confirmed by enrichment of the known marker enzyme activities in the isolated cellular fractions and almost complete recovery of the co-expressed cytochrome b₅ (Table 1).

**Table 1:**

| Marker enzyme activities in subcellular fractions of *Escherichia coli* | | | |
|---|---|---|---|
| Enzyme/protein | Activity/amount (% of total) | | |
| | Periplasm | Cytosol | Membranes |
| alkaline phosphatase | 94 | 5 | 1 |
| malate dehydrogenase | 12 | 2 | 86 |
| succinate dehydrogenase | 2 | 7 | 91 |
| fumarase | 5 | 90 | 5 |
| isocitrate dehydrogenase | 14 | 82 | 4 |
| cytochrome b₅ | 8 | 92 | 0 |

*E. coli* N4830-1 harbouring pYPS was thermo-induced at 38.5°C for 4 hours. The enzyme activities and the relative content of cytochrome b₅ were determined as described previously [16].

The cellular pool of PrSSU appeared enriched in the envelope membranes (Figure 2A,B, lane E+). The PrSSU protein proved undetectable in the periplasmic and the cytoplasmic fractions (Figure 2A,B, lanes P+, PD+ and C+). The thermoinduced profile of the envelope membranes revealed another dominant, co-expressed 17 kDa polypeptide that did not cross-react with the anti-SSU serum. The anti-SSU reactive 14 kDa protein localised in the periplasmic fraction (Figure 2B, lanes P+ and PD+) was of a size similar to pea stromal SSU (Figure 2A,B, cf lanes P+ and S). Moreover, the determined N-terminal sequence of the first five amino acid residues (MQVWP) matched with the mature SSU sequence.

To test the possibility that the recombinant PrSSU may have been accumulated in the cytoplasm of the intact bacterium in the form of inclusion bodies which co-isolated with the membranes during subcellular fractionation, a detailed time-course analysis was conducted by electron microscopy of the thermo-induced *E. coli* pYPS in comparison with the cell-line pλ-1cyt (Gallagher *et at.* (1992), Applied Microbiology and Biotechnology 38, 77-83) expressing cytochrome b₅ but not PrSSU. This study showed that both strains had normal ultracytomorphology throughout the induction phase of up to 8 hours (data not presented). Morphologically, these two recombinant strains were indistinguishable in their cell shape, size and distribution of the nucleoid. The absence of cytoplasmic protein aggregates discounted the likelihood of PrSSU protein being accumulated in the form of inclusion bodies. Unequivocal evidence for the localisation of PrSSU protein in the envelope zone was obtained by immuno-gold labelling of whole *E. coli* PYPS cell ulstrasections.

Whilst the immuno-gold labelling clearly showed that the PrSSU protein was targeted to the envelope zone, the approach did not indicate whether it was enriched in the IM or OM. To gain further insight, the cell envelope traction of thermo-induced *E. coli* pYPS was further resolved into the IM and OM fractions by discontinuous sucrose gradient centrifugation of the total membrane fraction, obtained by mild lysozyme digestion of EDTA-treated generated spheroplasts (Osborn *et al* (1972) Journal of Biological Chemistry 247,3962-3972). The envelope fraction resolved into two discrete bands with buoyant densities of r = 1.23±0.02 for the lower white band (OM) and 1.14±0.03 for the upper brown band (IM), values in close agreement with those previously reported. Greater than 85% of the total succinate dehydrogenase activity in the inner membrane and characteristic polypeptide profiles displayed by the two types of membranes (Figure 3A) substantiated that effective subfractionation of the two membranes had occurred. In the latter, the major of the outer membrane proteins OmpA, F and C appeared as the prominent bands but were absent in the IM. Comparison of the immunoelectrophoretogram in Figure 3 clearly shows that the envelope-localised PrSSU was discretely segregated in the OM and was undetectable in the IM. Detectable build-up of PrSSU in the isolated OM occurs at a longer (6h) induction duration (Figure 3) than that seen at 4h in the crude envelope fraction (Figure 2A,B, lane E+), possibly due to loss of some of the precursor protein during the lengthy subfractionation procedures. Localisation of PrS SU protein to the OM is concomitantly coupled with induction of two additional proteins of 15 kDa and 17 kDa. These proteins are not cross-reactive with anti-SSU sera and do not appear in the control thermoinduced cell line pλ-1cyt.

That the PrSSU protein was tightly integrated into the OM was indicated by the inability to extract it from the isolated membranes by washings with either 0.1 M Na₂CO₃ or 1 M NaCl (data not presented), treatments known to release loosely-bound and peripheral proteins. To seek whether the PrSSU protein was laterally exposed to the exterior of the cells, Sh thermoinduced, non-permeabilised, *E. coli* pYPS cells were treated with trypsin. Whilst, this 'shaving' approach specifically depleted a 35 kDa band, the PrSSU band remained unaffected, implying that it was most likely not exposed to the exterior to be susceptible to the exogenous protease (data not presented).

The above experiments demonstrate targeted expression of the pea PrSSU protein to the OM in *E. coli.* To some extent, the protein is also processed *en route* to generate a counterpart size related to the mature form in the periplasm. Following cytoplasmic synthesis and removal of the N-terminal methionine, PrSSU was rapidly targeted to the OM. Translocation to the OM could occur either directly from the cytosol via the contact zones or in consecutive steps through the IM and periplasm. The former pathway could account for the PrSSU absence in the IM whereas the latter pathway could explain the presences of the processed derivative in the periplasm. In view of the recent findings that the auxiliary periplasmic molecular chaperones are involved in the transit of unfolded IM-translocated OM proteins to the OM (Matsuyama *et al.* (1995), The EMBO Journal 14, 3365-3372), it is possible that the chromosomally co-expressed 15 kDa and 17 kDA proteins may be involved in the targeting pathway (at least in the case of the export of PrSSU).

### Example 2

### Expression and targeting of cytochrome b₅ to the OM in Escherichia coli

The SSU portion in the higher plant precursor gene was substituted with the mammalian globular cytochrome b₅ gene in the pYPS vector. The DNA encoding the 99 amino acid residue globular haemoprotein was placed in a direct reading frame with the transit peptide of SSU (Figure 1B). The results of this expression study were similar to those observed with the PrSSU. A significant proportion of processed globular cytochrome b₅ was localised in the periplasm and the chromogenic chimeric transit peptide-cytochrome b₅ was also targeted to the OM where it was retained as an integral, correctly folded holoprotein as indicated by its spectral properties (see Figures 4 and 5). The OM-targeted transit peptide-cytochrome b₅ displayed indistinguishable spectral characteristics in comparison with the native cytochrome b₅, including the Soret absorption peak at 423nm and the visible peaks at 555nm and 527nm. Thus, the arg⁻¹⁵ deleted transit peptide carries targeting information for localisation of a passenger protein to the OM of *E. coli.*

Most chloroplast transit peptides are particularly rich in hydroxylated amino acids and contain at least several evenly distributed, basic residues (Keegstra (1989), Cell 56, 247-253). Hence, they are considered to be more soluble in an aqueous environment than the corresponding hydrophobic secretory signal sequences. However, hydropathy analysis of the arginine-deleted transit peptide reveals two hydrophobic regions in the transit peptide, a shorter portion at the amino terminus (I) and a longer middle segment (II). Albeit displaying a lower hydrophobicity index, the intragenic region II comprising of 21 residues displays characteristics similar to signal sequences found in the OM proteins of *E. coli* (Figure 6). These features include an N-terminal region carrying a positively-charged residue, a central hydrophobic core and a C-terminal segment which contains a proline residue located some six residues from a plausible cleavage site according to the -3, -1 rule of von Heijne (Heijne (1990), Journal of Membrane Biology, 195-201). Similarly, Neilson and co-worker's *Signalp* program (Nielsen *et aL* (1 997) Protein Engineering 10, 1-6) predicts a potential signal sequence in the transit peptide portion spanning from 1-21 residues with a potential cleavage site between residues 21 and 22: QSA-AY. Such an N-terminal cleavage or non-cleavable signal sequence segment of the pea transit peptide could act as a membrane insertion loop to initiate the translocation of the passenger polypeptide, possibly by the *sec*-dependent translocation apparatus. Some of the subsequently translocated PrSSU may have undergone proteolysis to yield a 'trimmed' form related to the mature SSU in the periplasm. Precisely, how the transit peptide subsequently partitions into the OM remains to be elucidated and the presence of an additional sorting signal cannot be ruled out.

Although the particular transit peptide used in the above experiments has a deletion between domains I and II of an arginine that is highly conserved in most higher plants, the PrSSU transit peptides of *Silene pratensis* (Swiss-prot entry Q42516) and *Amaranthus hypochondriacus* (Swiss-prot entry Q42516) are also devoid of this basic residue. Moreover, the arginine-deleted mutant PrSSU is also import-competent into isolated pea chloroplasts (data not presented).

### Example 3

### Targeting of active human cytochrome p4501a1 (cyp1a1) to the periplasmic space of Escherichia coli

Native human cytochrome P4501A1 (CYP1A1) was appended at its amino terminus to the secretory signal *of Escherichia coli* alkaline phosphatase. The chimeric P450 construct was placed under the transcriptional control of the native *phoA* promoter in a prokaryotic expression vector. Induction of the hemoprotein by heterologous expression in *E. coli* following growth in a phosphate-limited medium resulted in abundant synthesis of recombinant CYP1A1 as detected by reduced CO-difference spectra. Furthermore, the signal-appended CYP1A1 was translocated across the bacterial inner membrane by the *sec*-dependent pathway and processed to yield authentic, heme-incorporated P450 within the periplasmic space. *In vitro* and whole-cell metabolic activity studies showed that the periplasmically-located CYP1A1 competently catalysed NADPH-dependent benzo[a]pyrene 3-hydroxylation and 7-ethoxyresorufin O-deethylation. The means to localise cytochromes P450 in the periplasm offers an ability to produce high levels of protein, attributable to the less hostile nature of the compartment and therein the enzymes for post-translational assembly of heme with the translocated protein.

Cytochromes P450 (CYP) are a superfamily of enzymes that are widely distributed in various forms of life including bacteria, plants, fungi, insects and mammals and which perform many important biological oxidations [1]. CYPs contain an iron protoporhyrin IX prosthetic group which catalyses the cleavage of bound molecular oxygen and results in the formation of an oxygenated product [2]. A plethora of biological reactions are catalysed by CYPs including highly selective regio- and stereospecific hydroxylations resulting in commercially relevant products including pharmaceutical and pesticide precursors [3,4]. Its unique catalytic chemistry is well known and has been exploited in whole-cell biotransformations, for example, the hydroxylation of corticosteroids by fungi [5]. However, improvement of biological systems to fully exploit CYPs for the synthesis of highly specific chemicals and detoxifying environmental pollutants remains a clear challenge in the areas of biocatalysis and bioremediation.

Currently, bacterial expression of microsomal human CYPs is generally undertaken intracellularly, often localising in the membrane through the CYP N-terminal membrane anchor. Expression in *Escherichia coli* often necessitates the modification of the initial part of the open-reading frame to optimise or allow production, therefore producing non-authentic protein. Others have reported the use of *PeIB* and *OmpA* leader sequences for CYP production, but successful translocation and processing was not observed [6,7]. In some cases soluble derivatives have also been produced that have allowed the first structural information on these proteins to be obtained after successful production of protein crystals [8], previously hindered by the hydrophobic N-terminal anchor. For the useful production and study of CYPs, higher yields of proteins are desirable, authentic protein production and circumventing permeability problems associated with the cell wall barrier in *E. coli.* Here we utilise the *phoA* promoter and leader sequence to successfully transport CYP1A1 to the periplasmic space in *E. coli,* where an extremely high yield was obtained. Catalytic activity of the protein was observed in an NADPH-dependent manner reflecting the endogenous electron donor system(s) present in this cellular compartment.

### Materials and Methods

2.1 Bacteria and plasmids. The *E.coli* strains used were DH5αF' [*F'*/*endA1 hsdr17* (rₖ⁻mₖ⁺) *supE44 thi-1 recA1 gyrA96(Nal*^{*r*}*) relA1 Δ (lacZYA-argF) U169 deoR* ϕ80*dlaΔ(lacZ)M15*] and TB-I *[F, araΔ(lac-proAB)rps* φ *80d lacZΔM*15*hsdR*17 (rₖ⁺ m⁺ₖ)]. Bacteria were propagated in Luria broth composed of 1%(w/v) tryptone (Difco), 0.5%(w/v) yeast extract (Difco), 0.5%(w/v) sodium chloride and supplemented with 100µg/ml ampicillin. CYP1A1 production was induced by growth of *E. coli* in phosphate-limited (0.1 mM) MOPS medium [9] in the presence of 100µg/ml ampicillin at 37°C for specified periods; a saturated LB-culture was used as a starter inoculum at 2%(v/v).

2.2 DNA manipulations. Plasmid DNA was isolated by the Wizard Midiprep DNA purification system from Promega. The standard procedures for DNA restriction, phosphorylation, agarose-gel electrophoresis, preparation of competent cells and transformation of competent *E. coli* cells were performed as described by Maniatis *et al.,* [10]. Polymerase Chain Reaction (PCR) was performed on an Hybaid OmniGene thermal cycler, using SuperTaq (Kramel Biotechnology, Cramlington, Northumberland, U.K.). Synthetic oligonucleotides were purchased from MWG Biotech (Milton Keynes, U.K.). Plasmid pCK1, which contains the cDNA for *CYP1A1,* was used as the template to amplify and manipulate *CYP141*. The gene was amplified as a PCR fragment of approximately 1500 bp containing the engineered *Hind*III and *Pst*I sites at 5' and 3' ends, respectively, using the following forward and reverse primers: forward primer 5'- GCTCAAGCTTCAATGGCTTTCCCAATCTCC -3'; reverse primer 5' -GCGCTGCAGCTAAGAGCGCAGCTGCATT-3'. The PCR conditions were as described previously[11]. The resulting *CYP1A1* DNA fragment was double digested with *Hind*III and *Pst*I and ligated into the *Hind*III and *Pst*I-cut pLiQ *E. coli* expression plasmid to produce pLiCYP1A1. The restriction and DNA modifying enzymes were purchased from Promega (Southampton) and their conditions for use followed as recommended by the supplier.

2.3 Subcellular fractions. Unless otherwise stated, all procedures were carried out at 4°C. Bacteria induced for heterologous expression (500ml) were harvested after 20h by centrifugation at 1500 × g for 10min. Periplasmic fractions were prepared by an 'osmotic shock' method as follows; the cells were plasmolysed by suspension in 20ml 20%(w/v) sucrose, 0.3M Tris-HCl (pH8), 1mM EDTA (STE buffer) and incubated at 22°C for 10min. Cells were harvested and resuspended in residual STE and osmotically shocked by rapid immersion in 2ml of ice chilled 0.5 mM MgCl₂. After incubation on ice for 10min, the periplasmic fraction was recovered by centrifugation at 15000 × g for 10min. The pellet was retained to provide the material for the preparation of cytoplasmic and membrane fractions. The residual cells, resuspended in 10ml 50mM Tris-HCl (pH 8), 5mM Na₂EDTA containing 10mg lysozyme were lysed by incubating on ice for 30min and sonication. To reduce the gelatinous chromosomal DNA, the lysate made up to 10mM MgCl₂, was further incubated for 30min with 0.2mg/ml DNase I. Centrifugation at 100000 × g for 30min separated the cytoplasm from the membranes which were prepared as described previously [9].

2.4 Spectrophotometric assays. Light-absorption spectra were measured using a Hitatchi U3010 scanning spectrophotometer. Extracts containing P450 were diluted in 100 mM KPi (pH 7.4) containing 1mM EDTA and 20% (v/v) glycerol. P450 concentration was estimated from CO reduced difference spectra according to Omura and Sato, [12], using an extinction coefficient of 91 mM⁻¹ cm⁻¹.

2.5 Reconstitution of enzyme activities. *In vitro* enzyme activities were reconstituted in a reaction mixture (1 ml final volume) consisting of the appropriate *E coli* periplasmic and cytoplasmic fractions containing 200 pmol of recombinant CYP1A1. *In vivo* enzyme activities of 1ml of *E. coli* culture harboring the control and CYP1A1 expression plasmids were assessed for enzymatic activity following incubation with substrate. 7-Ethoxyresorufin O-deethylation was estimated flurometrically, using an extraction procedure as described previously [13]. Benzo[a]pyrene 3-hydroxylation was measured fluorometrically according to the method ofNebert and Gelboin [14].

2.6 Other procedures and assays. Protein concentration was estimated using the bicinchoninic acid (BCA, Sigma) assay with bovine serum albumin as standard. SDS-polyacrylamide gel electrophoresis employed the discontinuous buffer system of Laemmli, [15]. Western blotting was carried out according to the method of Guengerich *et al.,* [16]. Western immunoblots were developed with polyclonal antibody against CYP1A1 (purchased from Gentest Corporation, CA, USA) using alkaline phosphatase for detection.

### 3. Results

3.1 Expression of CYP1A1 in the periplasmic fractions of *E. coli.* We report here an expression system for the targeting of cytochrome P450 to the periplasmic space of *E. coli* using the plasmid pLiCYP1A1. Previous studies for the successful expression of eukaryotic CYPs in *E. coli* have required modification at the N terminus of the P450 or require a leader sequence 5' to the P450 cDNA. In the present study, the *CYP1A1* cDNA was placed under the tight transcriptional control of the *phoA* promoter and expression induced by growth of the bacteria in a phosphate-limited medium. Lysates of induced bacteria displayed the characteristic reduced CO-difference absorption spectrum with a spectral maximum of 447 nm for the expressed native CYP1A1. The progressive increase in Soret absorbance with culture growth indicated that the intensifying red colour of the induced bacteria was derived from *de novo* synthesis of CYP.

3.2 CYP1A1 targeted to the periplasm *of E. coli.* Subcellular localisation of the recombinant CYP1A1 was investigated and bacteria were subfractionated into periplasmic, cytoplasmic and membrane fractions, with each fraction assayed for CYP content. CYP1A1 was efficiently expressed in the periplasm. At best, 4500 nmol CYP1A1/L culture was obtained compared with 25 nmol/L culture following N-terminal modification and expression in membranes as described previously [11]. The effective subcellular targeting to the periplasm was supported by the >90% enrichment of alkaline phosphatase (periplasm) and by checking different fractions for malate dehydrogenase (membranes) and fumarase (cytosol). More than 80% of the total cellular CYP1A1 content was found localised in the periplasmic fraction of *E. coli.* Further substantiation of the cellular location of CYP forms were sought by subjecting the periplasmic protein fractions derived from *E. coli* TB1 expressing CYP1A1 to SDS polyacrylamide gel electrophoresis and Western blotting. The identity of the full-length protein was verified by Western blot analysis probed with specific anti-CYP1A1 antibodies.

3.3 Periplasmically targeted CYP1A1 is enzymatically active *in vitro* and *in vivo* The catalytic activities of heterologously expressed CYP1A1 were measured *in vitro* and in whole cell biotransformations. The *in vitro* activities of CYP1A1 in the isolated periplasmic fractions were determined as a function of varying amounts of the extract The enzymatic rates of the protein were proportional to the amount of extract used in the assays. No activity was recorded in control samples derived from *E. coli* TB1 harbouring the empty plasmid, pLiQ. The data obtained demonstrated that the activity of native CYP1A1 was comparable to previous reports for these activities of *E. coli* membranes heterologously expressing CYP1A1, but which contained modification of the NH₂-terminal domain to allow expression [11]. The ability of the isolated periplasmic fractions to sustain the biotransformation capailities of targeted CYP1A1 in the present study strongly suggests that this compartment contains suitable P450 electron donor proteins; previously recombinant mammalian CYPs produced within *E. coli* could be supported by endogenous ferredoxin and ferredoxin reductase [17,18]. It appears that endogenous redox partners are also present in sufficient quantity to allow the high-level of expressed CYP to be functional within the periplasm without the need for co-expression or addition of the native counterparts. This validated the approach as a suitable one in order to obtain the highest yield of heterologous CYP1A1 in the literature coupled with an active protein in an advantageous accessible cellular location.

### 4. Discussion

In the present study several interesting and novel features of *E. coli* periplasmic space as an ideal location for targeting recombinant, human CYP(s) have emerged. Previously, the heterologous expression of human cytochromes P450 have employed many systems including bacterial [19], yeast [20], and COS cells [21]. The normal methods for bacterial expression of membrane-bound mammalian cytochromes P450, as pioneered by Barnes *et al.,* [19], involved the modification of the initial coding triplets. Subsequently, several groups have examined expression of human cytochromes P450 using the *PeIB* and *Omp*A leader sequences together with the *tac* promoter [6,7]. However, their experiments did not produce removal of the leader sequence such that non-authentic recombinant P450 was formed. Furthermore, movement of the cytochrome P450 into the periplasmic space was not demonstrated and hemoprotein yields were typical of previous studies producing a few hundreds nmol CYP/L bacterial culture at best. Recently, cytosolic expression has been reported in the 2-3000 nmol/L range by incubating cells in the presence of sub-toxic levels of chloramphenicol [22], but not to the 4500nmollL observed here using the *phoA* promoter.

The periplasm is a more oxidising environment than the cytosol, but results here show it provides a stable environment for accumulation of significant amounts of correctly folded and heme-associated CYP. The reason for the significantly high level of production of functional CYP may lie in the present strategy of targeting the protein to the periplasmic space, where the appropriate post-translational enzymic machinery is localised for heme incorporation. 5-aminolevulinic acid is the first committed precursor in *E. coli* tetrapyrrole biosynthesis [23]. It can be derived from the C-5 (glutamate) pathway, more commonly, and a route involving the condensation of succinyl CoA and glycine [24]. Biosynthesis from 5-aminolevulinic acid to iron protoporphyrin IX (protoheme = heme b) occurs in the cytosol involving the proteins coded for by *hemB-G.* In a subsequent step, formation of heme-thiolate proteins such as CYP requires insertion of the protoheme into the pocket of the apoprotein together with the formation of the fifth axial thiolate ligand of the CYP cysteine residue. Information on how this occurs is poorly understood. As *E. coli* does not produce CYP it would appear protoheme incorporation may occur spontaneously or autologously. Cytochrome of the c type, containing covalently bound heme c are widely distributed in nature where they function in photosynthetic and respiratory electron transfer chains [25]. *E. coli* does not contain a cytochrome bc₁ complex analogous to the mitochondrial electron transport chains components, but does contain a related cytochrome, cytochrome *c*_{*552*}, that contains six covalently bound heme groups and is located in the periplasm functioning as dissimilatory nitrate reductase [26]. The heme required for CYP production may be produced as for other cytochromes in this cellular compartment.

In *E. coli* no heme lyase activity or related coding gene are known and it is thought that cytochrome c assembly occurs spontaneously. Recently, *cydC* and *cydD* were identified, which are required for biogenesis of *c*-type cytochrome [27]. Either gene disruption prevented synthesis of cytochrome *bd* quinol oxidase. Both *cydC* and *cydD* are required for cytochrome c assembly and are also involved in heme transport to the periplasmic space where both types of cytochrome appear to be assembled. Dsb proteins also appear to be important in maintaining the thiol groups of the periplasmic apopoteins in a suitably reduced state. Again it remains to be seen whether formation of thiolate moiety of the CYP apoprotein requires such participation of the Dsb chaperones for assembly of CYP in the periplasm of *E. coli* [28].

We show here significant in vivo drug transformation capability of recombinant native CYP1A1 within *E. coli.* This CYP is a typical microsomal enzyme in eukaryotes, relying on NADPH-cytochrome P450 reductase for activity. Here an NADPH-dependent activity was found. Surprisingly, the periplasm of *E. coli* is aptly furnished with endogenous electron donors for *in vivo* transformation capability of the targeted CYP1A1. Consequently, the periplasmic location bypasses the permeability barrier of the inner membrane and thus promises wider applications of this heterologous P450 expression system for diverse industrial applications such as drug metabolite production for toxicological screening. Alternatively, it may be useful for bioremediation of environmental pollutants as CYP1A1 includes amongst its substrates many recalcitrant polycyclic aromatic hydrocarbons [29,30]. We also conclude that the periplasm of *E. coli* is an ideal compartment for targeting recombinant human CYP(s). This may be considered a preferential route to allow a high yield of microsomal CYPs, which may be useful in structural studies on this important superfamily of proteins.

### Example 4

### Export of cytochrome p450 105d1 to the periplasmic space of Escherichia coli

In this example, using methods generally as set out in Example 3, *Streptomyces griseus* CYP105D1 was appended at its amino terminus to the secretory signal of *Escherichia coli* alkaline phosphatase. The chimeric P450 construct was placed under the transcriptional control of the native *phoA* promoter in a prokaryotic expression vector. Induction of the hemoprotein by heterologous expression in *E. coli* following growth in a phosphate-limited medium resulted in abundant synthesis of recombinant CYP105D1 as detected by reduced CO-difference spectra. The signal-appended CYP105D1 was translocated across the bacterial inner membrane by a sec-dependent pathway and processed to yield authentic, heme-incorporated P450 within the periplasmic space. In *vitro* and whole cell metabolic activity studies showed that the periplasmically-located CYP105D1 competently catalysed NADH-dependent oxidation of the xenobiotic compounds benzo[a]pyrene and erythromycin, further revealing the presence in the *E. coli* periplasm of endogenous functional redox partners.

CYP 1 05D 1 has previously been expressed as a recombinant cytosolic form in *Escherichia coli* using the IPTG inducble *tac* promoter and enhanced drug transformation activities were observed in the enzyme preparations from the bacterial cell lysates (32). Due to selective permeability of *E. coli* to many substrates/ products, serious problems can arise in the optimisation of biotransformation processes using whole cell systems. For such reasons, cell wall mutants of *Salmonella typhimurium* were developed for use in mutagenesis tests (19). One approach to overcome these problems would be to engineer cytochromes P450 that can be exported to the periplasm or the cell exterior. In this experiment, we engineered a chimeric secretory form of *S. griseus cytochrome* P450 (CYP 105D1) that, when expressed in *E. coli,* leads to abundant synthesis of the precursor protein. The precursor was exported via the sec-dependent pathway to the periplasm where it was correctly processed and incorporated heme to yield a functional hemoprotein. We also show that periplasm of *E. coli* has the necessary endogenous redox partners to facilitate *in vivo* biotransformations which can have value for biocatalysis/ bioremediation strategies

### MATERIALS AND METHODS

### Bacteria and plasmids

The *E. coli* strains employed were those described in Example 3 and the bacteria were propagated and harvested under conditions substantially as set out in Example 3. In this example, the DH5α strain was used as a foster strain for introduction of *in vitro* ligated plasmid DNA whereas TB1 strain was subsequently employed for expression of the recombinant CYPI05D1.

The *CYP105D1* open-reading frame, cloned in the expression vector pLiQ, was induced by growth of *E. coli* in phosphate-limited (0.1mM) MOPS medium (30) in the presence of 100µg ampicillin /ml at 30°C for specified periods; a saturated LB-culture was used as a 2% (v/v) starter inoculum.

### DNA manipulations

These were carried out according to the general methods set out in Example 3. Genomic DNA, isolated from a 100 ml culture of *Streptomyces griseus* as described previously by Trower *et al.* (34), was used as the template to amplify *CYP105D1.* The gene was amplified as a PCR fragment of 1239 bp containing the engineered *Hind*III and *Pst*I sites at 5' and 3' ends, respectively, using the following forward and reverse primers:

forward primer; 5' – AACTGCAGATGACGGAATCCACGACGGAC – 3'
reverse primer; 5' – ATGATATCTCACCAGGCCACGGGCAGGT – 3'.

The PCR conditions were as described above. The resulting *CYP105D1* DNA fragment was doubly digested with *Hind*III and *Pst*I and ligated into the previously *Hind*III and *Pst*I-cut pLiQ *E. coli* expression plasmid. The restriction and DNA modifying enzymes were purchased from Promega (Southampton) and their use followed as recommended by the supplier.

### E. coli cultivation, harvesting and subcellular fractionations were carried out as in Example 3.

### Cytochrome P450 estimations

CYP content in biological samples were monitored by oxidised versus reduced difference spectroscopy of CO-bound hemoproteins according to Omura and Sato (25) using the molar absorption coefficient of 91000 M⁻¹ cm⁻¹. Extracts containing P450 were diluted with 100mM potassium phosphate buffer (pH 7.4) containing 1mM Na₂ EDTA and 20% (v/v) glycerol and light-absorption spectra measured using a Hitatchi U3010 scanning spectrophotometer.

### Enzyme assays

CYP105D1 enzyme activities were monitored in a 1ml final reaction volume composed of 200pmol of recombinant CYP105D1 (contained in an appropriate volume of *E. coli* periplasmic fraction or cells). The reaction was initiated by addition of NADH (1mM final concentration). For assessing enzyme activity in whole cells of induced *E. coli* TB1 harbouring either the control (pLiQ) or recombinant plasmid (pLiCYP105) following, the harvested cells (5000g x 2min) were washed twice with 0.1M potassium phosphate buffer (pH 7.4) containing 20% (v/v) glycerol and finally resuspended in 1ml of the same buffer for incubation with substrate. Erythromycin N-demethylation activity was determined as described previously (4). Benzo[a]pyrene 3-hydroxylation activity was measured fluorometrically using aPerkin Elmer fluorescence spectrphotometer according to the method ofNebert and Gelboin (22).

### Protein estimation and analysis

The protein content in bacterial fractions was estimated using the bicinchoninic acid (Sigma Chemicals) assay using bovine serum albumin as standard. Protein patterns were analysed by sodium dodecyl sulfate polyacrylamide gel electrophoresis (SDS-PAGE) employingthe discontinuous buffer system of Laemmli (13); approximately 75 µg of protein was loaded per lane.

### RESULTS

### Construction and expression of CYP105D1

This Example describes the development of an expression system for efficient targeting of cytochrome P450 to the periplasmic space of *E. coli* strain TB1 using the plasmid pLiQ.. This expression vector is a derivative of the previously described pAA-cyt (11), re-engineered with appropriate restriction sites downstream of the alkaline phosphatase signal sequence. Previous studies for the successful expression of cytochromes P450 in *E. coli* have required modification at the 5' end of the P450 gene or necessitated installation of a leader sequence *(PelB)* 5' to the P450 cDNA (3, 27). In the present study, the CYP105D1 cDNA was directly fused with the signal sequence and placed under the tight transcriptional control of the *phoA* promoter, inducible by growth of the bacteria in a phosphate-limited medium (30). Whole lysates derived from *E. coli* induced for 20h to express protein displayed the characteristic P450 CO-difference absorption spectra with defined spectral maxima of the Soret absorption at 448 nm that is a distinctive hallmark for cytochrome P450s (Figure 2). The progressive increase in the Soret absorbance peak with culture growth accompanied the intensifying red colour of the bacteria that was visually recoverable in the periplasmic fractions. This indicated derivation from *de novo* synthesis of a CYP-related hemoprotein. Interestingly, no significant peaks at around 420nm (an inactive form P450) in the freshly prepared cell-free extracts were observed prior to peak induction periods. This is in contrast to that reported for the production of the recombinant cytoplasmic counterpart (32) and suggested stable and functional production of CYP105D1.

### CYP105D1 is targeted to the periplasm of E. coli

A time-course profile of the production of CYP105D1 in the isolated periplasmic fractions was undertaken by estimating the hemoprotein content by difference spectroscopy (Figure 3). This study revealed that detectable periplasmic build-up of CYP105D1 occurs around 8h and steeply rises to a peak point around 25h from the start of growth. Following this period the levels of the cytochrome rapidly decline. The extent of the CYP105D1 production is indeed dramatic reaching a peak value exceeding 600 nmoles per litre of culture following addition of δ-aminolevulinic acid as a heme precursor. In some experiments >1000nmol of CYP105D1 have been obtained from this expression system. This level of hemoprotein is well in excess of the published figures for *E. coli* expression of cytochrome P450 in general. Previous studies reporting the heterologous expression of CYP105D1 under the control of the strongly inducible *tac* promoter obtained levels of CYP105D1 hemoprotein just over 400 nmol of P450 recovered in cytosol /litre culture when *E. coli* was cultured in the presence of 1mM δ-ALA (32). The present results indicated that heme could be successfully incorporated into P450 during folding of matured protein translocated into the periplasmic space of *E*. *coli.* In order to further investigate the subcellular localisation of the recombinant CYP, the bacteria induced for expression of the protein for 20h were subfractionated into periplasmic, cytoplasmic and membrane fractions. That effective subcellular fractionation had occurred was noted by >90% enrichment of the marker enzyme activity associated with the isolated subcellular fraction: alkaline phosphatase (periplasm), malate dehydrogenase (membranes) and fumarase (cytosol). More than 80% of the total cellular CYP105D1 content was found localised in the periplasmic space of *E. coli.* Further substantiation of the cellular location of CYP forms were sought by subjecting the periplasmic protein fractions derived from *E. coli* TB1 expressing CYP105D1 to SDS polyacrylamide gel electrophoresis. The results show induction of a novel protein of the expected size of ~45,000 D. Moreover, the polypeptide profile of the periplasmic fraction derived from *E. coli* expressing CYP105D1 show dramatic changes in the overall polypeptide composition when compared with counterpart control harbouring a plasmid without CYP105D1 gene. The most significant change is the intense co-overproduction of a 30 kDa protein whose identity was confirmed as β-lactamase through N-terminal protein sequence of the gel isolated protein band. A periplasmic fraction derived from *E. coli* pLiCYP105D1 following extensive dialysis againstwaterwas subjected to electron spray analysis. Molecular weights parsed around the expected region of CYP105D1 deconvoluted a major protein species with a molecular weight of 45406 Da, 4 Da lower than the size determined from the sequence of the cloned gene placed downstream of the secretory sequence. This verified that the exported protein was correctly processed by signal peptidase following its translocation across the inner membrane of *E. coli.*

Periplasmically targeted CYP105D1 is enzymically active in *in vivo* and *ex vivo* The catalytic activities of recombinant CYP105D1 was measured *in vivo* using whole cell biotransformation procedure and *in vitro* with isolated periplasmic fractions expressing CYP105D1.No activity was recorded in control samples derived from *E.* coli TB1 harbouring the empty plasmid pLIQ. The data obtained demonstrated that the activity of periplasmic targeted CYP105D1 was comparable to previous reports for such activities (32). The ability of the isolated fraction to sustain the xenobiotic transformation in *E. coli* periplasmic fraction strongly suggests that this compartment contains suitable electron donor proteins for P450 in the periplasmic space of *E. coli;* the native CYP 105D1 requires a ferredoxin and ferredoxin reductase. It appears that the foreign redox partners are also present in sufficient quantity to allow the high-level of expressed protein to be functional without the need for co-expression or addition of the native counterparts. This validated the approach as a suitable one in order to obtain high yields of heterologous CYP105D1 coupled with an active protein in a novel cellular location.

### Discussion

The heterologous expression of cytochromes P450 have employed many systems including bacterial (2), yeast (24), baculovirus (1) and COS cells (35). Reasons supporting this interest are described in the introduction and include involvement of CYP in current and potential industrial biotransformations as well as production and characterisation of proteins central to the toxicological fate of organic xenobiotics. The normal method for bacterial expression of membrane-bound eukaryotic cytochromes P450 involves the extensive modification of the N-terminus, although the soluble CYP105D1 did not require this for expression in the cytosol (32). Expression of cytochromes P450 using the *PeI*B and *Omp*A leader sequences have been examined together with the *tac* promoter (3,27). These previous studies did not reveal removal of the leader sequence or demonstrate movement of the cytochrome P450 into the periplasm and yields were typical of previous studies producing a few hundred nmol CYP/L culture. Recently, cytosolic expression of CYP 17 has been reported in the range from 2000 to 3000 nmol P450/lit culture by incubation of cells in the presence of sub-toxic levels of chloramphenicol (20). We also saw similar levels in the system described here on some occasions.

In this study several interesting and novel features of *E. coli* periplasmic space as an ideal site for targeting recombinant CYP have emerged which merit further discussion. Firstly the periplasm although a more oxidising environment than the cytosol provides a stable environment for accumulation of significant amounts of correctly folded and heme-associated holo-CYP over extended periods of cultivation. Secondly, drug transformation capability of native CYP105D1 is intrinsically dependent upon two additional redox partners, namely a ferredoxin and the cognate ferredoxin reductase. Surprisingly, the periplasm of *E. coli* is aptly furnished with the endogenous electron donors for *in* vivo transformation capability of the targeted CYP105D1. This together with broad substrate range of CYP105D1 and a location that bypasses the permeability barrier of the inner membrane indicates a potential for wider applications of the heterolous expression system for diverse industrial applications.

The reason for the significantly high level of production of functional holoCYP may lie in the present strategy of targeting the protein to the periplasm where the appropriate post-translational enzymic machinery is localised for heme assembly. In *E. coli* 5-aminolevulinic is the first committed precursor in tetrapyrrole biosynthesis (18). There are two routes for the derivation of 5-aminolevulinic acid, the C-5 (glutamate) pathway, which is now thought to be more common in the biosphere, and a route involving the condensation of succinyl CoA and glycine (6). The complex steps in biosynthesis from 5-aminolevulinic acid to iron protoporphyrin IX (protoheme = home *b)* occurs in the cytosol involving a battery of enzymes coded for by *hemB-G.* In a subsequent step, formation of hem-thiolate proteins such as CYP require insertion of the protoheme into the pocket of apoprotein together with fifth axial bridging via thiolate of cysteine residue. Since *E. coli* is not naturally endowed with synthesis of a CYP, or for that matter related heme-thiolate proteins, it would appear protoheme incorporation must occur spontaneously or autologously. Cytochrome of c type, which contain covalently bound heme c (derived by ligation of the vinyl groups to cysteine residues on the apoprotein) are widely distributed in nature where they function in photosynthetic and respiratory electron transfer chains (20). Although E. *coli* is capable of aerobic respiration it lacks soluble or a cytochrome bc₁ complex analogous to the mitochondrial electron transport chains components. The major related cytochrome *c in E. coli* is *cytochrome c*_{*552*} that contains six covalently bound heme groups and located in the periplasm, where it functions as dissimilatory nitrate reductase (10). In contrast to eukaryotes, no heme lyase activity or related coding gene has been identified in *E. coli* and so it so it is thought that cytochrome c assembly occurs spontaneously. Recently two *E. coli* genes *cydC and cydD* whose products are specifically required for biogenesis of c-type cytochrome have been identified (26). Disruption of either of these genes prevents the synthesis of cytochrome *bd* quinol oxidase. The *cydC* and *cydD* are proteins not only required for cytochrome c assembly but are also involved in heme transport to the periplasmic space where both types of cytochrome appear to be assembled. More recent findings implicate the role of Dsb proteins in maintaining the thiol groups of the periplsmic apopoteins in a suitably reduced state for assembly with heme *b* prosthetic group (21). It remains to be seen whether thiolate moiety of the apoprotein requiring the fifth axial co-ordination with the heme *b* also requires such participation of the Dsb chaperones in the assembly of CYP in the periplasm of *E. coli.* We therefore conclude that the periplasm of *E.coli* an favourable compartment for targeting recombinant cytochrome P450.

### References for Example 3

[1] Guengerich, F.P. (1991) Reactions and significance of cytochrome P-450 enzymes *J. Biol. Chem.* **266**, 10019-10022.
[2] Ortiz de Montellano, P.R., Ed. (1995) Cytochrome P450, 2^{nd} ed., Plenum, New York.
[3] Waterman, M.R., and Johnson, E.F., Eds. (1991) Methods in Enzymology, Vol. 206, Cytochrome P450, Academic Press, San Diego.
[4] Porter, T.D., and Coon, M.J. (1991) Cytochrome P450 - multiplicity of isoforms, substrates and catalytic and regulatory mechanisms. *J. Biol. Chem.* 266, 13469-13472
[5] Suzuki, K., Sanga, K., Chikaoka, Y. and Itagaki, E. (1993) Purification and properties of cytochrome P450 (P450(lun)) catalysing steroid 11β-hydroxylation in *Curvularia lunata. Biochem. Biophys. Acta.* **1203**, 215-223.
[6] Blake, J.A.R., Pritchard, M., Ding, S.H., Smith, G.C.M., Burchell, B., Wolf, C.R., and Friedberg, T. (1996) Coexpression of a human P450 (CYP3A4) and P450 reductase generates a highly functional monooxygenase system in *Escherichia coli. FEBS Lett.* 397, 210-214.
[7] Pritchard, M.P., Ossetian, R, Li, D.N., Henderson, C.J., Burchell, B., Wolf, C.R., and Friedberg, T. (1997) A general strategy for the expression of recombinant human cytochrome P450s in *Escherichia coli* using bacterial signal peptides: Expression of CYP3A4, CYP2A6 and CYP2E1 *Arch Biochem. Biophys.* 345,342-354.
[8] Williams, P.A., Cosme, J., Sridhar, V., Johnson, E.F., and McRee, D.E. (2000) Mammalian microsomal cytochrome P450 monooxygenase; structural adaptions for membrane binding and functional diversity. *Mol. Cell* **5**,121-131.
[9] Liu Y-Y., Akhtar M.K., Ourmodzi E.P., Kaderbhai N. and Kaderbhai M.A. (2000) A chloroplast envelope-transfer sequence functions as an export signal in *Eschaerichia coli. FEBS Lett.* **469**, 61-66.
[10] Maniatis, T., Fritsch, E.F., and Sambrook, J. (1989) *Molecular Cloning. A Laboratory Manual.* Cold Spring Harbor Laboratory, Cold Spring Harbor, New York.
[11] Guo, Z., Gillam, E.M.J., Ohmori, S., Tukey, R.H., and Guengerich, F.P. (1994) Expression of modified human cytochrome P4501A1 in *Escherichia coli* - Effects of 5' substitution, stabilization, purification, spectral characterisation and catalytic properties. *Arch. Biochem. Biophys.* **312**,436-446.
[12] Omura, T., and Sato, R. (1964) The carbon monoxide binding pigment of liver microsomes. I. Evidence for its hemeoprotein nature. *J. Biol. Chem.* **239**, 2370-2378.
[13] Burke, M.D., and Mayer, R.T. (1975) Inherent specificities of purified cytochromes P450 and P448 towards biphenyl hydroxylation and ethoxyresorufin deethylation. *Drug Metab. Dispn.* **3**,245-253.
[14] Nebert, D.W., and Gelboin, H.V. (1968) Substrate inducible microsomal aryl hydroxylase in mammalian cell culture. I. Assay and properties of the induced enzyme. *J. Biol. Chem.* **243,** 6242-6249.
[15] Laemmli, U.K. (1970) Cleavage of structural proteins during the assembly of the head of bacteriophage T₄. *Nature* **227**, 680-685.
[16] Guengerich, F.P., Wang, P., and Davidson, N.K. (1982) Estimation of isozymes of microsomal cytochrome P450 in rats, rabbits and humans using immunochemical staining coupled with sodium dodecyl sulfate polyacrylamide gel electrophoresis. *Biochemistry* **21*,*** 1698-1706.
[17] Jenkins, C.M., and Waterman, M.R. (1994) Flavodoxin and NADPH flavodoxin reductase from *Escherichia coli* support bovine cytochrome P450C17 hydroxylase activities. *J. Biol. Chem.* **269:** 27401-27408.
[18] Jenkins, C.M., and Waterman, M.R. (1998) NADPH flavodoxin reductase and flavodoxin from *Escherichia coli;* Characteristics as a soluble microsomal P450 reductase. *Biochemistry* **37:** 6106-6113.
[19] Barnes, H.J., Arlotto, M.P. and Waterman, M.R. (1991) Expression and enzymatic activity of recombinant cytochrome P450 17α-hydroxylase in *Escherichia coli. Proc. Natl. Acad. Sci. (U.S.A).* **88**, 5597-560I.
[20] Oeda, K., Sakaki, T., and Ohkawa, H. (1985) Expression of rat liver cytochrome P450MC cDNA in *Saccharomyces cerevisiae. DNA* **4**,203-210.
[21] Zuber, M.X., Simpson, E.R., and Waterman, M.R. (1986) Expression of bovine 17α-hydroxylase cytochrome P450 cDNA in nonsteroidogenic (COS-1) cells. *Science* **234**, 1258-1261.
[22] Kusano, K., Waterman, M.R., Sakaguchi, M., Omura, T., and Kagawa, N. (1999) Protein synthesis inhibitors and ethanol selectively enhance heterologous expression of P450s and related proteins in *Escherichia coli. Arch Biochem. Biophys.* **367**, 129-136.
[23] Matters, G.L., and Beale, S.I. (1994) Biosynthesis of δ-aminoleluvinic acid from glutamate by *Sulfolobus solfataricus. Arch. Microbiol.* **161**, 272-276.
[24] Castelfranco, P.A., and Beale, S.I. (1983) Chlorophyll biosynthesis-recent advances and areas of current interest. *Arm. Rev.Plant Physiol. Plant Mol. Biol.* **34**, 241-278.
[25] Meyer, T.E. and Cusanovich, M.A. (1989). Structure, function and distribution of soluble bacterial redox proteins. *Biochim. Biophys. Acta.* **975**, 1-28.
[26] Kajie, S.-I. and Anraku, Y. (1986) Purification of a hexahemecytochrome C552 from *Escherichia coli* K12 and its properties as a nitrite reductase. *Eur. J: Biochem.* ***154,*** 457-463.
[27] Poole, R.K, Hatch, L., Cleeter, M.W.J., Gibson, F., Cox, G.B., Wu, G.H. (1993) Cytochrome BD biosynthesis in *Escherichia coli* - the sequences of the CYDC and CYDD genes suggest that they encode the components of an ABC membrane transporter. *Mol. Microbiol.* **10***,* 421-43 0.
[28] Missiakas, D. and Raina, S. (1997) Protein folding in the bacterial periplasm. *J. Bacteriol.* **179,** 2465-2471.
[29] Shimada, T., Yun, C-H., Yamazaki, H., Gautier, J-C., Beaune, P.H., and Guengerich, F.P. (1992) Characterisation of human lung microsomal cytochrome P4501A1 and its role in the oxidation of chemical carcinogens. *Mol. Pharmacol.* **41,** 856-864.
[30] McManus, M.E., Burgess, W.M., Veronese, M.E., Huggett, A., Quattrochi, L.C., and Tukey, R.H. (1990) Metabolism of 2-acetylaminofluorene and benzo[a]pyrene and activation of food derived heterocyclic amine mutagens by human cytochromes P450. *Cancer Res.* **50,** 3367-3376.

### References for Example 4

1. **Asseffa, A., S. Smith, J. Gillette, H. V. Gelboin, and F. J. Gonzalez.** 1989. Novel exogenous heme-dependent expression of mammalian cytochrome P450 using baculovirus. Arch. Biochem. Biophys. 274:481-490.
2. **Barnes, H. J., M. P. Arlotto, and M. R. Waterman.** 1991. Expression and enzymatic activity of recombinant cytochrome P450 17α-hydroxylase in *Escherichia coli.* Proc. Natl. Acad. Sci. U.S.A. **88**:5597-5601.
3. **Blake, J. A. R., M. Pritchard, S. H. Ding, G. C. M. Smith, B. Burchell, C. R. Wolf, and T. Friedberg.** 1996. Co-expression of a human cytochrome P450 (CYP3A4) and P450 reductase generates a highly functional monooxygenase system in *Escherichia coli.* FEBS Lett. **397**:210-214.
4. **Brian, W. R., M. A. Sari, M. Iwasaki, T. Shimada, L. S. Kaminisky, and F. P.** Guengerich. 1990. Catalytic activities of human liver cytochrome P450IIIA4 expressed in *Saccharomyces cerevisiae.* Biochemistry **305**:11280-11292.
5. **Cannell R. J. P., A. R. Knaggs, M. J. Dawson, G. R. Manchee, P. J. Eddershaw, I. Waterhouse, D. R. Sutherland, G. D. Bowers, and P. J. Sidebottom.** 1995. Microbial biotransformation of the angiotensin II antagonist GR117289 by *Streptomyces rimosus* to identify a mammalian metabolite. Drug. Metab. Disp. **23**:724-729.
6. **Castelfranco, P. A., and S. I. Beale.** 1983. Chlorophyll biosynthesis - recent advances and areas of current interest. Annu. Rev. Plant Phys. **34**:241-278.
7. **Guengerich, F.P.** 1991. Reactions and significance of cytochrome P450 enzymes J. Biol. Chem. **266**:10019-10022.
8. **Hanahan, D.** 1983. Studies on transformation of *Escherichia coli* with plasmids. J. Mol. Biol. **166**:557-580.
9. **Harding, V., N. Kaderbhai, A. Karim, A. Evans, A. Jones, and M. A. Kaderbhai.** 1993. Processing of chimeic mammalian cytochrome b₅ precursors in *Escherichia coli* - reaction specificity of signal peptidase and identification of an aminopeptidase in post-translocational processing. Biochem. J. **293**:751-756.
10. **Kajie, S.-I. and Y. Anraku.** 1986. Purification of a hexaheme cytochrome C552 from *Escherichia coli* K12 and its properties as a nitrite reductase. Eur. J. Biochem. **154**:457-463.
11. **Karim, A., N. Kaderbhai, A. Evans, V. Harding, and M. A. Kaderbhai.** 1993. Efficient bacterial export of a eukaryotic cytoplasmic cytochrome. Biotechnology **11**:612-618.
12. **Kusano, K., M. R. Waterman, M. Sakaguchi, T. Omura, and N. Kagawa.** 1999. Protein synthesis inhibitors and ethanol selectively enhance heterologous expression of P450s and related proteins in *Escherichia coli.* Arch, Biochem. Biophys. **1**:129-136.
13. **Laemmli, U. K.** 1970. Cleavage of structural proteins during the assembly of the head of bacteriophage T₄. Nature **277**:680-685.
14. **Lee-Robichound, P., M. A. Kaderbhai, N. Kaderbhai, N. J. Wright, and M. Akhtar.** 1997. Interaction of human CYP17 (P450_{17Υ}, 17α-hydroxylase-17,20-lyase) with cytochrome b₅ : importance of the orientation of the hydrophobic domain of cytochrome b₅. Biochem: J. **321**:857-863.
15. **Liu, Y-Y., M. K. Akhtar, E. P. Ourmodzi, N. Kaderbhai, and M. A. Kaderbhai.** 2000. A chloroplast envelope transfer sequence functions as an export signal in *Escherichia coli.* FEBS Lett. **469**:61-66.
16. **Liu,Y-Y., N. Kaderbhai, and M. A. Kaderbhai.** 2000. A mammalian cytochrome fused to a chloroplast transit peptide is a functional hemoprotein and is imported into isolated chloroplasts. Biochem. J. **351**:377-384.
17. **Maniatis, T., E. F. Fritsch, and J. Sambrook.** 1989. *Molecular Cloning. A Laboratory Manual.* Cold Spring Harbor Laboratory, Cold Spring Harbor, New York.
18. **Matters, G. L., and S. I. Beale.** 1994. Biosynthesis of δ-aminoleluvinic acid from glutamate by *Sulfolobus solfataricus.* Arch. Microbiol. **161**:272-276.
19. **McCann J., E. Choi, E. Yamasaki, and B. N. Ames.** 1975. Proc. Nat. Acad. Sci. (USA) **72**:5135-5139.
20. **Meyer, T. E. and M. A. Cusanovich. 1989.** Structure, function and distribution of soluble bacterial redox proteins. Biochim. Biophys. Acta **975**:1-28.
21. **Missiakas, D. and S. Raina. 1997.** Protein folding in the bacterial periplasm. J. Bacteriol. **179**:2465-2471.
22. **Nebert, D. W., and H. V. Gelboin.** 1968. Substrate inducible microsomal aryl hydroxylase in mammalian cell culture. I. Assay and properties of induced enzyme. J. Biol. Chem. **243**:6242-6249.
23. **Nebert, D. W., D. R. Nelson, M. J. Coon, R. W. Estabrook, R. Feyereisen, Y.** Fujiikuriyama, F. J. Gonzalez, F. P. Guengerich, I. **C. Gunsalus,** E. F. Johnson, **J. C. Loper, R. Sato, M. R. Waterman, and D. J. Waxman.** 1990. The P450 superfamily - update on new sequences, gene mapping and recommended nomenclature. DNA Cell Biol. **10**:1-14.
24. **Oeda, K., T. Sakaki, and H. Ohkawa.** 1985. Expression of rat liver cytochrome P450MC synthesised in *Saccharomyces cerevisiae.* DNA **4**:203-210.
25. **Omura, T., and R. Sato.** 1964. The carbon monoxide binding pigment of liver microsomes. I. Evidence for its hemoprotein nature. J. Biol. Chem. **239**:2370-2378.
26. **Poole, R. K, L. Hatch, M. W. J. Cleeter, F. Gibson, G. B. Cox, and G. H. Wu.** 1993. Cytochrome BD biosynthesis in *Escherichia coli -* the sequences of the CYDC and CYDD genes suggest that they encode the components of an ABC membrane transporter. Mol. Microbiol. **10**:421-430.
27. **Pritchard, M. P., R. Ossetian, D. N. Li, C. J. Henderson, B. Burchell, C. R. Wolf, and T. Friedberg.** 1997. A general strategy for the expression of recombinant human cytochrome P450s in *Escherichia coli* using bacterial signal peptides: Expression of CYP3A4, CYP2A6 and CYP2E1. Arch. Biochem. Biophys. **345**:342-354.
28. **Sariaslani, F. S., and D. A. Kunz.** 1986. Induction of cytochrome P450 in *Streptomyces griseus* by soybean flour. Biochem. Biophys. Res. Commun. **141**:405-410.
29. **Serizawa, N., and T. Matsuoka.** 1991. A two component type cytochrome P450 monooxygenase system in a prokaryote that catalyses hydroxylation of ML-236B to pravastatin, a tissue selective inhibitor of 3-hydroxy-3-methylglutaryl coenzyme A reductase. Biochem. Biophys. Acta **1084**:35-40.
30. **Shortie, D.** 1983. A genetic system for analysis of *Staphylococcal* nuclease. Gene **22**:181-189.
31. **Suzuki, K., K. Sanga, Y. Chikaoka, and E. Itagaki.** 1993. Purification and properties of cytochrome P450 (P450lun) catalysing steroid 11β-hydroxylation in *Curvularia lunata.* Biochem. Biophys. Acta **1203**:215-223.
32. **Taylor, M., D. C. Lamb, R. Cannell, M. Dawson, and S. L. Kelly.** 1999. Cytochrome P450105D1 (CYP105D1) from *Streptomyces griseus;* heterologous expression, activity and activation effects of multiple xenobiotics. Biochem. Biophys. Res. Commun. **263**:838-842.
33. **Trower, M. K., F. S. Sariaslani, and F. G. Kitson. 1988.** Xenobiotic oxidation by cytochrome P450 enriched extracts of *Streptomyces griseus.* Biochem. Biophys. Res. Commun. **157**:1417-1422.
34. **Trower, M. K., R. Lenstra, C. Omer, S. E. Buchholz, and F. S. Sariaslani.** 1992. Cloning, nucleotide sequence determination and expression of the genes encoding cytochrome P450_{soy} (soyC) and ferredoxin_{soy} (soyB) from *Streptomyces griseus.* Mol. Microbiol. **6:**2125-2134.
35. **Zuber, M.X., E. R. Simpson, and M. R Waterman.** 1986. Expression of bovine 17α-hydroxylase cytochrome P450 cDNA in nonsteroidogenic (COS-1) cells. Science **234**:1258-1261.

## Claims

1. A vector comprising nucleic acid encoding a stromal targeting domain (STD) operably linked to a heterologous gene encoding a polypeptide of interest, said vector further comprising prokaryotic expression elements for expressing the heterologous gene in a Gram-negative prokaryote and for targeting expression of the polypeptide of interest to the outer membrane and/or periplasmic space of the Gram-negative prokaryote.

2. Use of a vector, comprising nucleic acid encoding a stromal targeting domain (STD) operably linked to a heterologous gene encoding a polypeptide of interest, for expressing the heterologous gene in a Gram-negative prokaryote and targeting expression of the polypeptide of interest to the outer membrane and/or periplasmic space of the Gram-negative prokaryote.

3. A vector or use of a vector according to either claim 1 or 2, wherein the STD is comprised in a chloroplast transit peptide.

4. A vector or use of a vector according to any one of the preceding claims, wherein the polypeptide of interest is a haemoprotein, optionally wherein the haemoprotein is a member of the cytochrome P-450 superfamily of enzymes.

5. A vector or use of a vector according to any one of the preceding claims, wherein the vector comprises prokaryotic expression elements for directing expression in *Escherichia coli.*

6. A vector or use of a vector according to any one of the preceding claims, wherein the prokaryotic expression elements comprise a promoter and/or a ribosome binding site.

7. A vector or use of a vector according to any one of the preceding claims, wherein the vector further comprises nucleic acid encoding a periplasmic signal sequence, optionally wherein the signal sequence is the bacterial alkaline phosphatase signal sequence.

8. A vector or use of a vector according to claim 7, wherein the nucleic acid encoding the signal sequence is located upstream of that encoding the STD.

9. A vector or use of a vector according to any one of the preceding claims, wherein
(a) the vector further comprises:
(i) a multiple cloning site for inserting a gene encoding a polypeptide of interest into the vector; and/or
(ii) nucleic acid encoding one or more selectable marker(s) and/or reporter elements; and/or
(iii) one or more prokaryotic origin(s) of replication; and/or
(b) the vector is a plasmid.

10. A prokaryotic host cell comprising the vector used in any one of claims 1 to 9, optionally wherein the host cell is *Escherichia coli.*

11. A composition comprising the host cell of claim 10 for use as an inoculum, optionally wherein the composition further comprises a carrier, for example wherein the carrier is a cryoprotective agent, such as glycerol.

12. A process for producing a polypeptide of interest comprising: either
(i) the steps of: (a) culturing the host cell of claim 10, (b) harvesting the cultured cells; (c) fractionating the harvested cells to provide a fraction enriched in outer membranes; and (d) isolating the polypeptide of interest from the outer membrane fraction; or
(ii) the steps of: (a) culturing the host cell of claim 10; (b) harvesting the cultured cells; (c) fractionating the harvested cells to provide a periplasmic fraction; and (d) isolating the polypeptide of interest from the periplasmic fraction.

13. A process for producing a membrane-bound polypeptide of interest comprising the steps of (a) culturing the host cell of claim 10; (b) harvesting the cultured cells; (c) fractionating the harvested cells to provide a fraction enriched in outer membranes containing the membrane-bound polypeptide of interest.

14. A process according to either of claims 12 or 13 wherein
(i) step (a) comprises inoculating a growth medium with the composition of any one of claims 11; and/or
(ii) the preliminary step of introducing the vector of the invention into a Gram-negative prokaryote (for example, *Escherichia coli)* to provide the host cell of claim 10, optionally wherein the vector is a plasmid and is introduced into the host cell by transformation.

15. An industrial fermentation comprising the process of any one of claims 12 to 14.

## Patentansprüche

1. Vektor, der Nucleinsäure umfasst, die eine stromale Targeting-Domäne (STD) kodiert, die funktionell mit einem heterologen Gen verknüpft ist, das ein Polypeptid von Interesse kodiert, wobei der genannte Vektor ferner prokaryotische Expressionselemente zum Exprimieren des heterologen Gens in einem gramnegativen Prokaryoten und zum Richten der Expression des Polypeptids von Interesse auf die äußere Membran und/oder den periplasmatischen Raum des gramnegativen Prokaryoten umfasst.

2. Verwendung eines Vektors, der Nucleinsäure umfasst, die eine stromale Targeting-Domäne (STD) kodiert, die funktionell mit einem heterologen Gen verknüpft ist, das ein Polypeptid von Interesse kodiert, zum Exprimieren des heterologen Gens in einem gramnegativen Prokaryoten und Richten der Expression des Polypeptids von Interesse auf die äußere Membran und/oder den periplasmatischen Raum des gramnegativen Prokaryoten.

3. Vektor oder Verwendung eines Vektors nach Anspruch 1 oder 2, wobei die STD in einem Chloroplastentransitpeptid enthalten ist.

4. Vektor oder Verwendung eines Vektors nach einem der vorherigen Ansprüche, wobei das Polypeptid von Interesse ein Hämoprotein ist, das optional ein Mitglied der Cytochrom-P-450-Überfamilie von Enzymen ist.

5. Vektor oder Verwendung eines Vektors nach einem der vorherigen Ansprüche, wobei der Vektor prokaryotische Expressionselemente zur Lenkung der Expression in *Escherichia coli* umfasst.

6. Vektor oder Verwendung eines Vektors nach einem der vorherigen Ansprüche, wobei die prokaryotischen Expressionselemente einen Promotor und/oder eine Ribosomen-Bindungsstelle umfassen.

7. Vektor oder Verwendung eines Vektors nach einem der vorherigen Ansprüche, wobei der Vektor ferner eine Nucleinsäure umfasst, die eine periplasmatische Signalsequenz kodiert, wobei die Signalsequenz optional die bakterielle alkalische Phosphatase-Signalsequenz ist.

8. Vektor oder Verwendung eines Vektors nach Anspruch 7, wobei die Nucleinsäure, die die Signalsequenz kodiert, stromaufwärts von der liegt, die die STD kodiert.

9. Vektor oder Verwendung eines Vektors nach einem der vorherigen Ansprüche, wobei
(a) der Vektor ferner Folgendes umfasst:
(i) eine multiple Klonierungsstelle zum Einsetzen eines Gens, das ein Polypeptid von Interesse kodiert, in den Vektor; und/oder
(ii) Nucleinsäure, die einen oder mehrere selektierbare Marker und/oder Reporterelemente kodiert; und/oder
(iii) einen oder mehrere prokaryotische(n) Replikationsstartpunkt(e); und/oder
(b) der Vektor ein Plasmid ist.

10. Prokaryotische Wirtszelle, umfassend den in einem der Ansprüche 1 bis 9 verwendeten Vektor, wobei die Wirtszelle optional *Escherichia coli* ist.

11. Zusammensetzung, umfassend die Wirtszelle aus Anspruch 10 zur Verwendung als Inokulum, wobei die Zusammensetzung ferner optional einen Träger umfasst, der zum Beispiel ein Kälteschutzmittel wie Glycerol ist.

12. Verfahren zur Herstellung eines Polypeptids von Interesse, umfassend: entweder
(i) die folgenden Schritte: (a) Kultivieren der Wirtszelle aus Anspruch (10), (b) Ernten der kultivierten Zellen, (c) Fraktionieren der geernteten Zellen, um eine mit äußeren Membranen angereicherte Fraktion zu erhalten und (d) Isolieren des Polypeptids von Interesse von der äußeren Membranfraktion; oder
(ii) die folgenden Schritte: (a) Kultivieren der Wirtszelle aus Anspruch 10, (b) Ernten der kultivierten Zellen, (c) Fraktionieren der geernteten Zellen, um eine periplasmatische Fraktion zu erhalten und (d) Isolieren des Polypeptids von Interesse von der periplasmatischen Fraktion.

13. Verfahren zur Herstellung eines membrangebundenen Polypeptids von Interesse, umfassend die folgenden Schritte: (a) Kultivieren der Wirtszelle aus Anspruch 10, (b) Ernten der kultivierten Zellen, (c) Fraktionieren der geernteten Zellen, um eine mit äußeren Membranen angereicherte Fraktion zu erhalten, umfassend das membrangebundene Polypeptid von Interesse.

14. Verfahren nach Anspruch 12 oder 13, wobei
(i) Schritt (a) das Inokulieren eines Wachstumsmediums mit der Zusammensetzung aus einem der Ansprüche 11 umfasst; und/oder
(ii) den einleitenden Schritt der Einführung des erfindungsgemäßen Vektors in einen gramnegativen Prokaryoten (zum Beispiel *Escherichia coli),* um die Wirtszelle aus Anspruch 10 bereitzustellen, wobei der Vektor optional ein Plasmid ist und in die Wirtszelle durch Transformation eingeführt wird.

15. Industrielle Fermentation, umfassend das Verfahren aus einem der Ansprüche 12 bis 14.

## Revendications

1. Vecteur comprenant un acide nucléique codant pour un domaine de ciblage du stroma (DCS) lié opérativement à un gène hétérologue codant pour un polypeptide présentant un intérêt, ledit vecteur comprenant également des éléments d'expression procaryotes pour exprimer le gène hétérologue chez un procaryote Gram négatif et pour cibler l'expression du polypeptide présentant un intérêt sur la membrane extérieure et/ou l'espace périplasmique du procaryote Gram négatif.

2. Utilisation d'un vecteur, comprenant un acide nucléique codant pour un domaine de ciblage du stroma (DCS) lié opérativement à un gène hétérologue codant pour un polypeptide présentant un intérêt, pour exprimer le gène hétérologue chez un procaryote Gram négatif et pour cibler l'expression du polypeptide présentant un intérêt sur la membrane extérieure et/ou l'espace périplasmique du procaryote Gram négatif.

3. Un vecteur ou l'utilisation d'un vecteur selon soit la revendication 1, soit la revendication 2, dans lequel le DCS est compris dans un peptide de transit de chloroplaste.

4. Un vecteur ou l'utilisation d'un vecteur selon n'importe laquelle des revendications précédentes, le polypeptide présentant un intérêt étant une hémoprotéine, l'hémoprotéine étant optionnellement un membre de la superfamille d'enzymes à cytochrome P-450.

5. Un vecteur ou l'utilisation d'un vecteur selon n'importe laquelle des revendications précédentes, le vecteur comprenant des éléments d'expression procaryotes pour diriger l'expression dans *Escherichia coli*.

6. Un vecteur ou l'utilisation d'un vecteur selon n'importe laquelle des revendications précédentes, les éléments d'expression procaryotes comprenant un promoteur et/ou un site de liaison ribosomique.

7. Un vecteur ou l'utilisation d'un vecteur selon n'importe laquelle des revendications précédentes, le vecteur comprenant également un acide nucléique codant pour une séquence signal périplasmique, optionnellement la séquence signal étant la séquence signal de la phosphatase alcaline bactérienne.

8. Un vecteur ou l'utilisation d'un vecteur selon la revendication 7, dans lequel l'acide nucléique codant pour la séquence signal étant situé en amont de celui codant pour le DCS.

9. Un vecteur ou l'utilisation d'un vecteur selon n'importe laquelle des revendications précédentes,
(a) le vecteur comprenant également :
(i) un site de clonage multiple pour insérer un gène codant pour un polypeptide présentant un intérêt dans le vecteur ; et/ou
(ii) un acide nucléique codant pour un ou plusieurs marqueurs et/ou éléments reporteurs pouvant être sélectionnés ; et/ou
(iii) une ou plusieurs origines de réplication procaryote ; et/ou
(b) le vecteur est un plasmide.

10. Cellule hôte procaryote comprenant le vecteur utilisé dans n'importe laquelle des revendications 1 à 9, optionnellement la cellule hôte étant *Escherichia coli.*

11. Un composé comprenant la cellule hôte de la revendication 10 destiné à être utilisé comme inoculum, ce composé comprenant également optionnellement un véhicule, par exemple lorsque le véhicule est un agent cryoprotecteur comme le glycérol.

12. Procédé pour la production d'un polypeptide intéressant comprenant :
(i) soit les étapes suivantes : (a) culture de la cellule hôte de la revendication 10, (b) récolte des cellules cultivées, (c) fractionnement des cellules cultivées pour avoir une fraction enrichie en membranes extérieures et (d) isolement du polypeptide présentant un intérêt de la fraction de membranes extérieures ;
(ii) soit les étapes suivantes : (a) culture de la cellule hôte de la revendication 10, (b) récolte des cellules cultivées, (c) fractionnement des cellules cultivées pour avoir une fraction périplasmique et (d) isolement du polypeptide présentant un intérêt de la fraction périplasmique.

13. Procédé pour la production d'un polypeptide intéressant lié à une membrane comprenant soit les étapes suivantes : (a) culture de la cellule hôte de la revendication 10 ; (b) récolte des cellules cultivées, (c) fractionnement des cellules cultivées pour avoir une fraction enrichie en membranes extérieures contenant le polypeptide présentant un intérêt lié à la membrane.

14. Procédé selon l'une ou l'autre des revendications 12 ou 13 dans lequel
(i) l'étape (a) comprend l'inoculation d'un milieu de culture ayant la composition de n'importe laquelle des revendications 11 ; et/ou
(ii) l'étape préliminaire consistant à introduire le vecteur de l'invention dans un procaryote Gram négatif (par exemple *Escherichia coli)* afin d'avoir la cellule hôte de la revendication 10, optionnellement le vecteur étant un plasmide et étant introduit dans la cellule hôte par transformation.

15. Fermentation industrielle comprenant le procédé décrit dans n'importe laquelle des revendications 12 à 14.
